# EUROPEAN PATENT APPLICATION

(11) **EP 4 079 848 A1**
(43) Date of publication of application: **26.10.2022**
(21) Application number: 21169774.3
(22) Date of filing: 22.04.2021
(51) Int. Cl.: C12N 9/54, A61K 38/48, C12N 9/64

(54) **IMMUNOGLOBULIN CLEAVING ENZYME**

(71) Applicant: Genovis Ab, 220 07 Lund (SE)
(72) Inventor: The designation of the inventor has not yet been filed
(74) Representative: D Young & Co LLP

(57) **Abstract**

The present invention relates to a novel polypeptide which displays protease activity against immunoglobulins, particularly human IgG, and *in vivo* and *ex vivo* uses thereof. Uses of the polypeptide include methods for the analysis of IgG and the generation of antibody fragments, as well as methods for the prevention or treatment of diseases and conditions mediated by IgG.

## Description

### Field of the Invention

The present invention relates to a novel polypeptide which displays protease activity against immunoglobulins, particularly human IgG, and *in vivo* and *ex vivo* uses thereof. Uses of the polypeptide include methods for the analysis of IgG and the generation of antibody fragments, as well as methods for the prevention or treatment of diseases and conditions mediated by IgG.

### Background of the Invention

IdeS (Immunoglobulin G-degrading enzyme of *S. pyogenes,* also known as imlifidase) is an extracellular cysteine protease produced by the human pathogen *S. pyogenes.* IdeS has an extraordinarily high degree of substrate specificity, with its only identified substrate being IgG. IdeS catalyses a single proteolytic cleavage in the lower hinge region of the heavy chains of all subclasses of human IgG. IdeS also catalyses an equivalent cleavage of the heavy chains of some subclasses of IgG in various animals. IdeS efficiently cleaves IgG to Fc and F(ab')₂ fragments via a two-stage mechanism. In the first stage, one (first) heavy chain of IgG is cleaved to generate a single cleaved IgG (scIgG) molecule with a single non-covalently bound Fc chain. The scIgG molecule is effectively an intermediate product which retains the remaining (second) heavy chain of the original IgG molecule. In the second stage of the mechanism this second heavy chain is cleaved by IdeS to release a F(ab')₂ fragment and a homodimeric Fc fragment. These are the products generally observed under physiological conditions. The homodimeric Fc may dissociate into its component monomers. Under reducing conditions the F(ab')₂ fragment may dissociate to two Fab fragments. The IgG cleaving ability of IdeS has been shown to have utility *ex vivo,* for example in methods for production of Fab, F(ab')₂ and Fc fragments, which may be used *e.g.* for the analysis of IgG and *in vitro* generation of F(ab')₂ fragments. See, for example, WO2003051914 and WO2009033670. IdeS and IgG cleaving proteins developed by modification of IdeS have also been shown to have *in vivo* utility as therapeutic agents, see for example, WO2006131347, WO2013110946, WO2016012285, WO2016128558, and WO2016128559. Such proteins are useful in this context because they are capable of the *in vivo* cleavage of IgG molecules which mediate disease or which are otherwise undesirable. The removal of IgG can be beneficial because IgG contributes to the pathology of many autoimmune conditions as well as to acute rejection of transplanted organs, and may also contribute to anti-drug responses. Antibodies specific for a given therapeutic agent may reduce its efficacy. This is particularly the case for antibody-based therapeutics, gene therapy vectors, and cell therapies including adoptive cell transfer (ACT) immunotherapies e.g. using CAR-T cells. In other words, IdeS and IgG cleaving proteins developed by modification of IdeS may be used as a therapy for any disease or condition wholly or partly mediated by IgG, which condition may include anti-drug responses.

However, IdeS is a virulence factor of S. pyogenes, which is responsible for common infections like tonsillitis and strep throat. Accordingly, most human subjects have encountered IdeS in this context and are likely to have anti-IdeS antibodies in the bloodstream, which may reduce its utility as a therapeutic agent.

IdeZ is an IgG cysteine protease produced by *Streptococcus equi ssp. zooepidemicus,* a bacterium predominantly found in horses. IdeZ has approximately 66% identity to IdeS. Since *Streptococcus equi ssp. zooepidemicus* is not a human pathogen, IdeZ was considered to be an alternative to IdeS-based therapies because humans may have fewer or no antibodies (anti-drug antibodies, ADA) against IdeZ. However, IdeZ has a level of IgG cysteine protease activity against human IgG which is considerably lower than that of IdeS, in particular when cleaving IgG2.

Given the multiple applications for immunoglobulin cleaving enzymes outlined above, there is a clear need for additional agents which are capable of cleaving immunoglobulins, particularly human IgG.

### Summary of the Invention

The present inventors have identified, purified and characterised a novel polypeptide. The polypeptide was identified in a previously unknown streptococcal species isolated as part of a study in multiple rodents. One particular isolated species, tentatively called *Streptococcus krösus,* was sequenced and was found to express several glycosidases and proteases.

A polypeptide of approximately 56.4 kDa, consisting of 557 amino acids was identified and is referred to herein as IdeSORK. The full length sequence is shown in SEQ ID NO: 1. The full length sequence is relatively difficult to express. However, the inventors have found that N terminal fragments of the full length sequence have significantly enhanced expression, whilst preserving immunoglobulin cleaving activity.
For example, the sequence of SEQ ID NO: 2 consists of the 312 amino acids from the N terminus of SEQ ID NO: 1. A polypeptide consisting of a version of the sequence of SEQ ID NO: 2 which is engineered for better expression and purification (addition of N terminal methionine and a C-terminal protein purification tag - His6) is referred to herein as IdeSORK2.0, the sequence of which is shown in SEQ ID NO: 3.

Provided herein is:
A polypeptide, optionally an engineered polypeptide, having immunoglobulin protease activity, wherein said polypeptide comprises, consists essentially of, or consists of:
(a) the amino acid sequence of SEQ ID NO: 1;
(b) the amino acid sequence of SEQ ID NO: 2;
(c) an amino acid sequence which is an N terminal fragment of the sequence of SEQ ID NO: 1;
(d) an amino acid sequence which is at least 50% identical to the amino acid sequence of any one of (a), (b) or (c);
optionally wherein said polypeptide is engineered to include an additional methionine at the N terminus and/or a protein purification or other tag at the C terminus, which tag may be joined to the C terminus by a linker.

The polypeptide may be provided in a composition, typically comprising an effective amount of a preservative. The polypeptide may be immobilised.

The polypeptides of the invention are useful in various methods for cleaving immunoglobulins, typically IgG. Thus, provided herein are methods comprising administering a polypeptide of the invention, for example to a sample or a subject in which IgG is present. The method may be a method of cleaving an immunoglobulin, and may optionally further comprise the detection or analysis of the cleavage products. The method may be a method treating a disease, the method comprising administering a polypeptide of the invention to a subject.

### Brief Description of the Figures

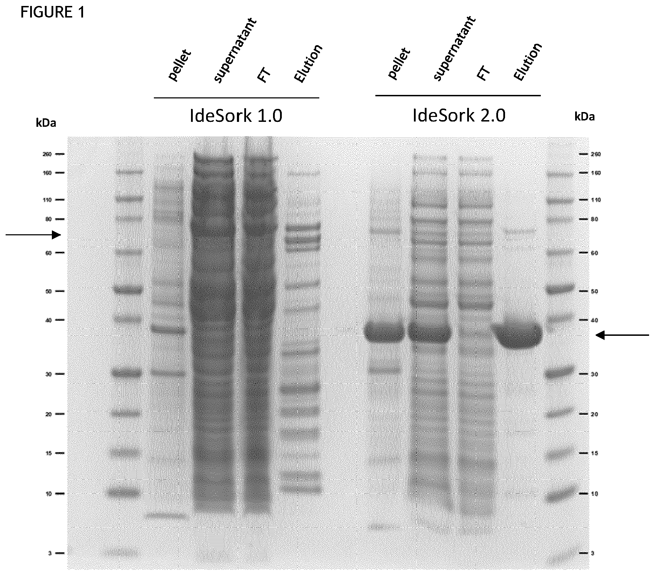

Figure 1: Expression and purification of IdeSork. The full length protein (1.0) is expressed as a ca 70 kDa protein, but expresses poorly. The C-terminally truncated IdeSork (2.0) expresses as a ca 36 kDa protein. Some material is insoluble (pellet) using the specified production protocol, but the vast majority can be solubilized (supernatant) and affinity-purified to high purity and yield.

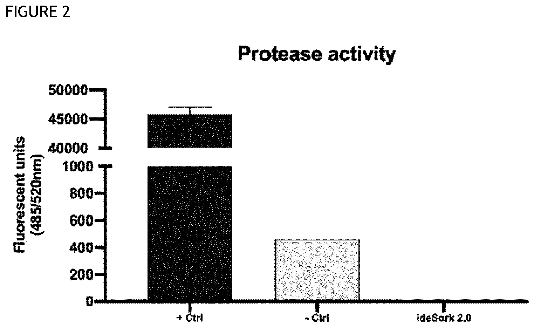

Figure 2. General protease activity. General protease activity was measured with the EnzCHECK protease assay, using casein as a substrate. The positive control FabULOUS is a general protease giving a high signal, while the negative control FabRICATOR is an IgG-specific protease, thus giving almost no signal. IdeSork 2.0 did not give any measurable signal in the assay, indicating a more specific enzyme.

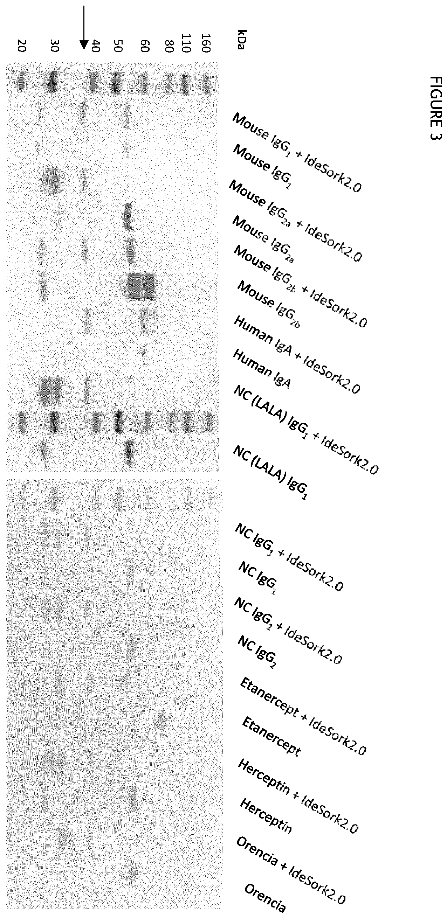

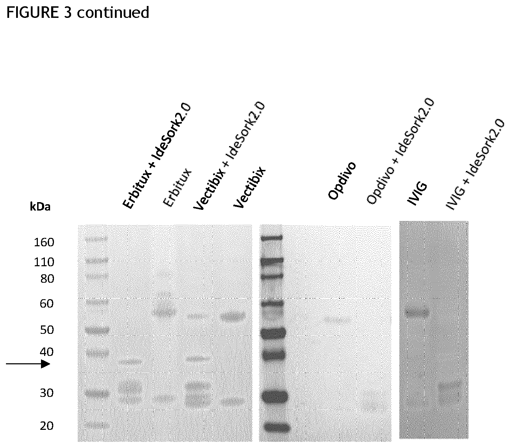

Figure 3. Specificity of IdeSork2.0. IdeSork2.0 was mixed with different antibodies and incubated o/n at 37°C before analysis on SDS-PAGE. The enzyme itself is noted with an arrow. All human IgG, or fusion proteins based on IgG, were hydrolyzed by IdeSork2.0, while only IgG2a from mouse could be hydrolyzed. Mouse IgG1, mouse IgG2b and human IgA were not affected by IdeSork2.0.

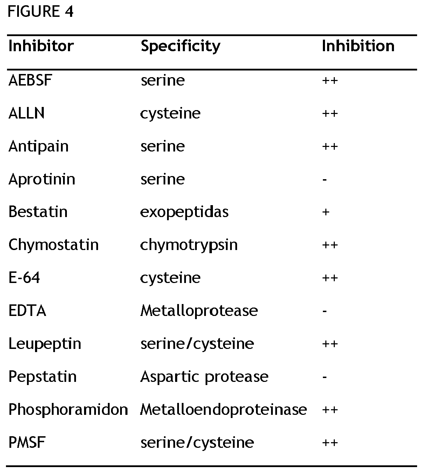

Figure 4. Protease inhibitor sensitivity of IdeSork2.0. IdeSork2.0 is inhibited by most protease inhibitors, besides aprotinin, EDTA, and pepstatin. Bestatin is also only partly inhibitory to the enzyme.

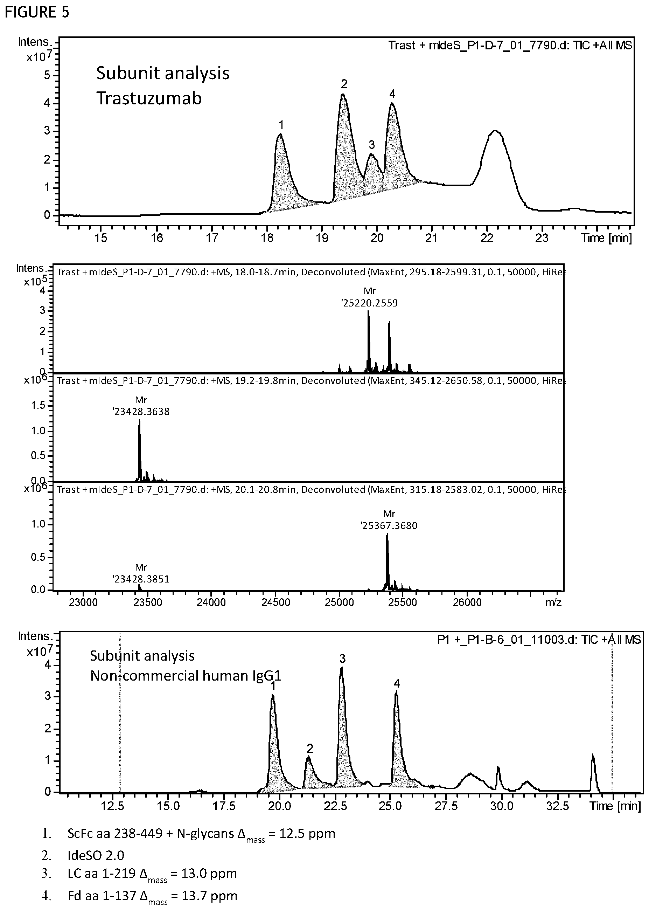

Figure 5. IdeSork2.0 hydrolyses the hinge of IgG at an identical site as IdeS. Commercial and non-commercial biologics were hydrolyzed with IdeSork2.0 and hydrolysis products were analyzed using LC-MS. In all cases hydrolysis was only observed at a single site in the hinge region, corresponding to the same site as IdeS recognizes.

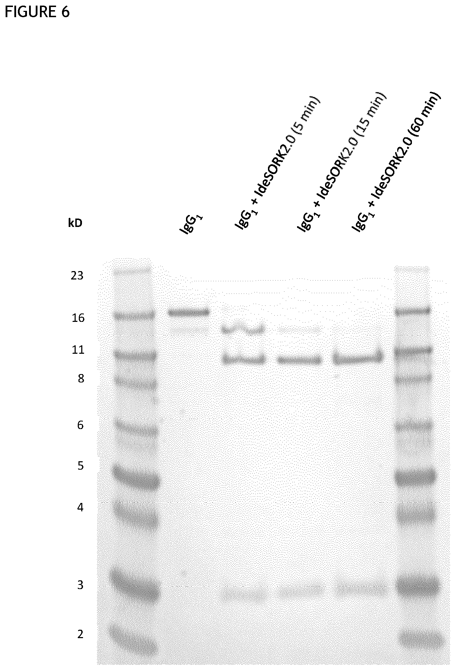

Figure 6. IdeSork2.0 is acting upon IgG in a two-step mechanism. To investigate if IdeSork2.0 is hydrolyzing both heavy chains of IgG simultaneously, or acting on them individually, we incubated Trastuzumab with IdeSork2.0 (1:20) and incubated for 0-60 minutes before analyzing on a non-reduced SDS-PAGE. Most IgG is hydrolyzed within the first few minutes, with one and two chains being hydrolyzed, indicating that it is a two-step reaction.

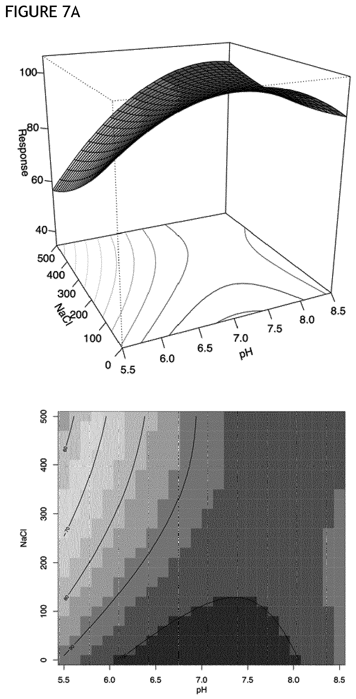

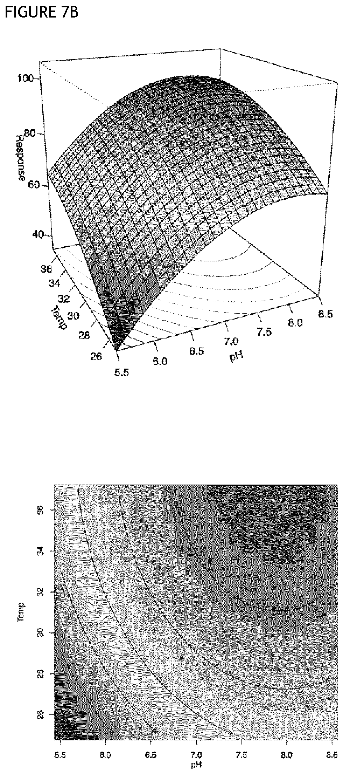

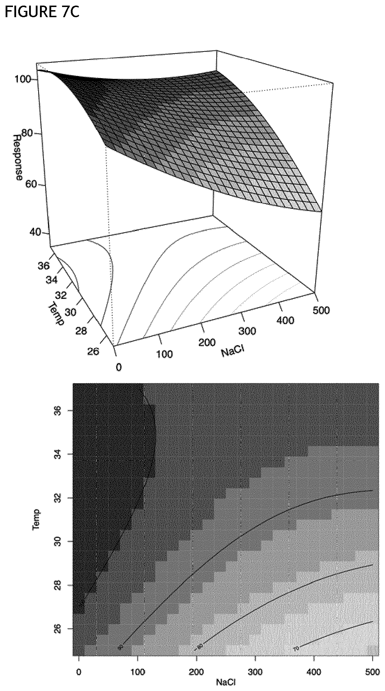

Figure 7. IdeSork2.0 optimal conditions. The optimal buffer composition for IdeSork2.0 was determined using a Box-Behnken experimental design, measuring the impact of pH, NaCl and temperature. Output was measures as percentage hydrolysis of IgG.

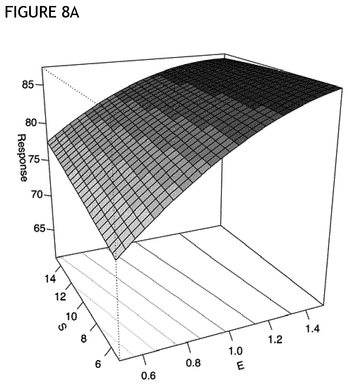

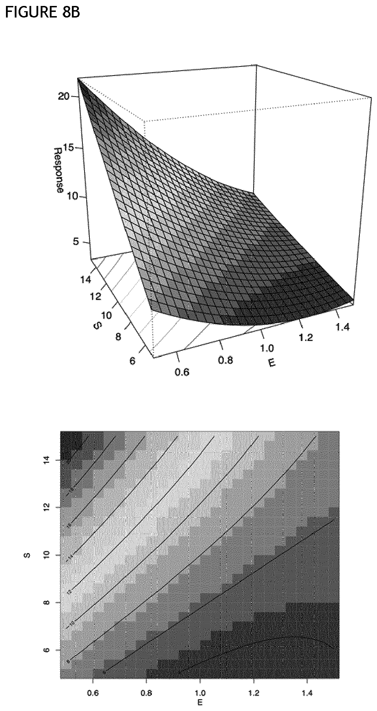

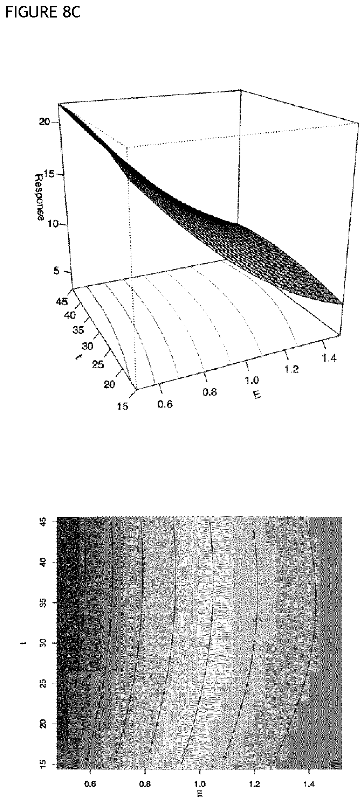

Figure 8. IdeSork2.0 enzyme:substrate ratio. The impact of enzyme and substrate concentration for the reaction speed was investigated using a Box-Behnken experimental setting, also analyzing the impact of time for the reaction speed. Output was measured as percentage hydrolysis of IgG (top left figure), as well as quantity hydrolyzed IgG per unit enzyme (all other figures).

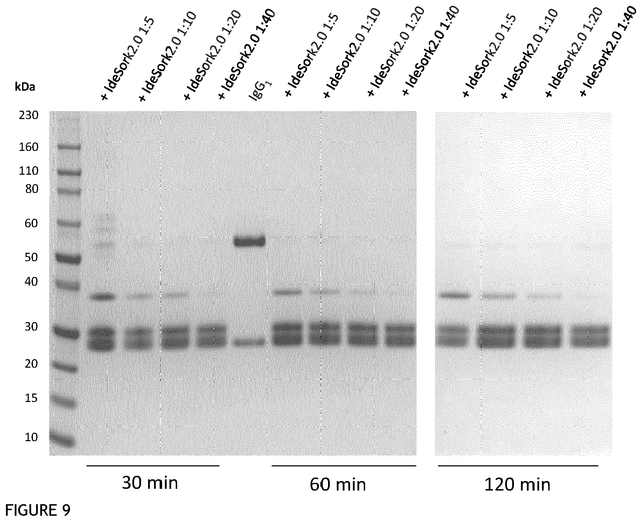

Figure 9. IdeSork2.0 enzyme: substrate activity over time. To verify the impact of modifying the enzyme:substrate ratio, an experiment with varying amounts of substrate (5-40 µg) to a set amount of enzyme (1 µg) was conducted over time (30, 60, and 120 min). Almost all IgG is hydrolyzed within 30 minutes at all conditions.

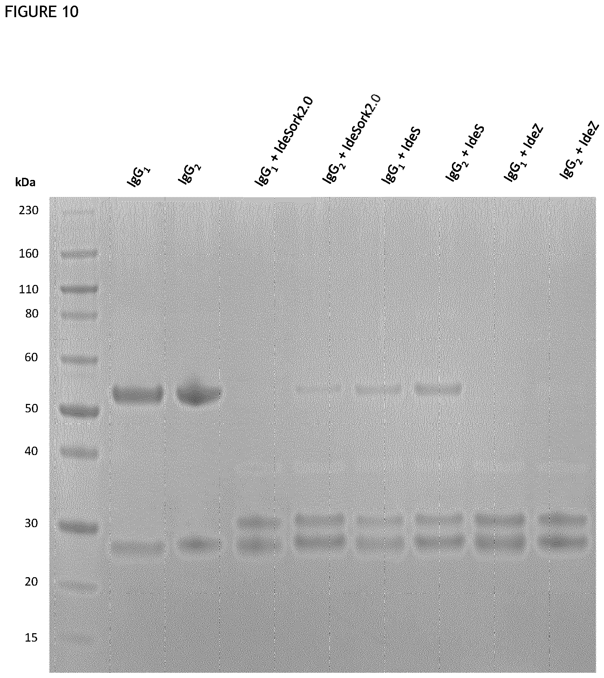

Figure 10. IdeSork2.0 is having a similar activity compared to IdeS and IdeZ. To investigate if IdeSork2.0 is hydrolyzing IgG in a similar way as IdeS and IdeZ they were all incubated against IgG1 and IgG2 and analyzed on SDS-PAGE. The cleavage patterns look identical, with both IdeSork2.0 and IdeS having less activity towards IgG2.

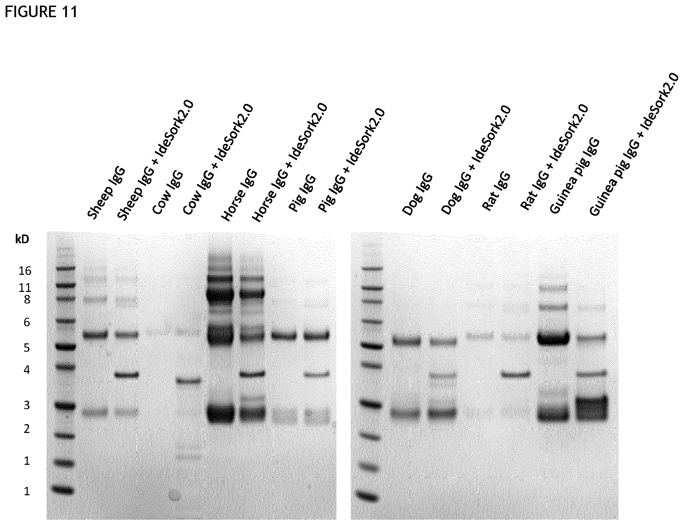

Figure 11. Specificity of IdeSork2.0. IdeSork2.0 was mixed with different antibodies and incubated o/n at 37°C before analysis on SDS-PAGE. IdeSORK2.0 had no visible effect on sheep, pig, dog, or rat IgG, but showed activity on horse and guinea pig IgG.

### Brief Description of the Sequences

SEQ ID NO: 1 is the full length amino acid sequence of a polypeptide isolated from a streptococcal species designated *Streptococcus krösus.* The polypeptide is 557 amino acids in length and has immunoglobulin protease activity. It may be referred to herein as IdeSORK
SEQ ID NO: 2 is an amino acid sequence of a polypeptide having immunoglobulin protease activity. The C-terminal of 245 amino acids of SEQ ID NO: 1 are deleted to form this sequence, which thus consists of the N terminal 312 amino acids of IdeSORK. This sequence may be described as a N terminal fragment of IdeSORK having immunoglobulin protease activity.
SEQ ID NO: 3 is an amino acid sequence of a polypeptide having immunoglobulin protease activity. This sequence consists of the sequence of SEQ ID NO: 2, engineered to include an additional N terminal methionine and a C-terminal protein purification tag - His₆. The resulting 319 amino acid polypeptide may be referred to herein as IdeSORK2.0.
SEQ ID NOs: 4 to 11 are the sequences of the Hinge/CH2 regions of various human and mouse IgG subclasses.
SEQ ID NO: 12 is the full length amino acid sequence of IdeS, which is publically available as NCBI Reference Sequence no. WP_010922160.1. This sequence includes an N terminal methionine followed by a 28 amino acid secretion signal sequence.
SEQ ID NO: 13 is the mature IdeS protein. The N terminal methionine and the signal sequence (a total of 29 amino acids at the N terminus) are removed from SEQ ID NO: 12 to form this sequence, which is also publically available as Genbank accession no. ADF13949.1. When used in the Examples, IdeS typically comprises this sequence engineered to include an additional N terminal methionine and a C-terminal protein purification tag, such as poly-histidine. A preferred tag is His₆.
SEQ ID NO: 14 is the full length amino acid sequence of IdeZ, which is publically available as NCBI Reference Sequence no WP_014622780.1. This sequence includes an N terminal methionine followed by a 33 amino acid secretion signal sequence.
SEQ ID NO: 15 is the mature IdeZ protein. The N terminal methionine and the signal sequence (a total of 34 amino acids at the N terminus) are removed from SEQ ID NO: 14 to form this sequence. When used in the Examples, IdeZ typically comprises this sequence engineered to include an additional N terminal methionine and a C-terminal protein purification tag, such as poly-histidine. A preferred tag is His₆.
SEQ ID NO: 16 is an exemplary nucleotide sequence encoding the polypeptide of SEQ ID NO: 1 engineered for expression with an N terminal histidine and a C terminal His₆.
SEQ ID NO: 17 is an exemplary nucleotide sequence encoding the polypeptide of SEQ ID NO: 2 engineered for expression with an N terminal histidine and a C terminal His₆. SEQ ID NO: 17 encodes SEQ ID NO: 3.
SEQ ID NO: 18 is an exemplary expression vector sequence comprising the sequence of SEQ ID NO : 16.
SEQ ID NO: 19 is an exemplary expression vector sequence comprising the sequence of SEQ ID NO : 17.

### Detailed Description of the Invention

It is to be understood that different applications of the disclosed products and methods may be tailored to the specific needs in the art. It is also to be understood that the terminology used herein is for the purpose of describing particular embodiments of the invention only, and is not intended to be limiting. All publications, patents and patent applications cited herein, whether supra or infra, are hereby incorporated by reference in their entirety.

As used in this specification and the appended claims, the singular forms "a", "an", and "the" include plural referents unless the content clearly dictates otherwise. Thus, for example, reference to "a polypeptide" includes "polypeptides", and the like.

### Polypeptides having immunoglobulin protease activity

### Functional features of a polypeptide having immunoglobulin protease activity

This section sets out the functional features of a polypeptide having immunoglobulin protease activity, which apply in addition to the structural features outlined in the immediately following section.

The present invention provides a polypeptide having immunoglobulin protease activity. That is, the polypeptide is able to cleave an immunoglobulin molecule. The immunoglobulin molecule is typically an IgG molecule, and preferably the polypeptide does not cleave other classes of immunoglobulin, The polypeptide may cleave any immunoglobulin molecule comprising a hinge/CH2 sequence of any one of SEQ ID NOs. 4 to 8 as shown in Table 1.

**Table 1**

| Subclass | Hinge/CH2 sequence | SEQ ID | Subclass | Hinge/CH2 sequence | SEQ ID |
|---|---|---|---|---|---|
| Human | | | Mouse | | |
| IgG1 | CPPCPAPELL**GG**PSVF | 4 | IgG1 | PCICTVPEVSSVF | 10 |
| IgG2 | CPPCPAPPV**AG**PSVF | 5 | IgG2a | CPPCAAPNLL**GG**PSVF | 8 |
| IgG3 | CPRCPAPELL**GG**PSVF | 6 | IgG2b | CHKCPAPNLEGGPSVF | 11 |
| IgG4 | AHHAQAPEFL**GG**PSVF | 7 | IgG3 | GSSCPAGNIL**GG**PSVF | 9 |

The polypeptide preferably cleaves between the bold/underlined residues as shown for each of these sequences in Table 1. The cleavage site may alternatively be described as being between positions 249 and 250 of human IgG according to the Kabat numbering system (positions 236 and 237 according to EU numbering system).

The polypeptide preferably cleaves all human IgG subclasses, that is IgG1, IgG2, IgG3 and IgG4, and does not cleave other human immunoglobulin classes. The polypeptide may exhibit lower activity against human IgG2 as compared to the other human IgG subclasses. The polypeptide may exhibit greater activity against human IgG1 than human IgG2. The polypeptide may exhibit activity against non-human IgG molecules, including mouse IgG2a and IgG3. The polypeptide may exhibit activity against IgG from guinea pig or horse.

The polypeptide efficiently cleaves IgG to Fc and F(ab')₂ fragments via a two-stage mechanism. In the first stage, one (first) heavy chain of IgG is cleaved to generate a single cleaved IgG (scIgG) molecule with a single non-covalently bound Fc chain. The scIgG molecule is effectively an intermediate product which retains the remaining (second) heavy chain of the original IgG molecule. The complex of a polypeptide of the invention and a single cleaved IgG molecule is also provided herein. In this context, the IgG cleaved by the polypeptide is preferably a recombinant, monoclonal antibody. Thus, a complex of a polypeptide of the invention and a single cleaved recombinant, monoclonal IgG molecule is provided. Exemplary antibodies that may be cleaved by the polypeptide of the invention are listed below in the "Methods of use" section. A complex of a polypeptide of the invention and a single cleaved version of any of these antibodies is provided.

In the second stage of the mechanism the remaining (second) heavy chain of the original IgG molecule is quickly cleaved by the polypeptide to release a F(ab')₂ fragment and a homodimeric Fc fragment. These are the products generally observed under physiological conditions. The homodimeric Fc may dissociate into its component monomers. Under reducing conditions the F(ab')2 fragment may dissociate to two Fab fragments.

Immunoglobulin protease activity may be assessed by any suitable method, for example by incubating a polypeptide with a sample containing IgG and determining the presence of IgG cleavage products. Assays for assessing immunoglobulin protease activity may also be used to quantify the efficacy of said activity, that is to assess the potency of a polypeptide. Efficacy may be assessed in the presence or absence of an inhibitor. However, efficacy herein will typically mean efficacy as assessed in the absence of such an inhibitor unless otherwise stated. Suitable assays to determine activity and/or quantify potency of said activity are well known in the art and any suitable assay may be used. Suitable assays include an ELISA-based assay. In such an assay, the wells of an assay plate will typically be coated with an antibody target, such as bovine serum albumin (BSA). Samples of the polypeptide to be tested are then added to the wells, followed by samples of target-specific antibody, that is antibody specific for BSA in this example. The polypeptide and antibody are allowed to interact under conditions suitable for IgG protease activity. After a suitable interval, the assay plate will be washed and a detector antibody which specifically binds to the target-specific antibody will be added under conditions suitable for binding to the target-specific antibody. The detector antibody will bind to any intact target-specific antibody that has bound to the target in each well. After washing, the amount of detector antibody present in a well will be proportional to the amount of target-specific antibody bound to that well. The detector antibody may be conjugated directly or indirectly to a label or another reporter system (such as an enzyme), such that the amount of detector antibody remaining in each well can be determined. The higher the potency of the tested polypeptide that was in a well, the less intact target-specific antibody will remain and thus there will be less detector antibody. Typically, at least one well on a given assay plate will include mature IdeS and/or mature IdeZ and/or IdeSORK2.0 instead of a polypeptide to be tested, so that the potency of the tested polypeptides may be directly compared to the potency of mature IdeS and/or mature IdeZ and/or IdeSORK2.0.

Other assays may determine the potency of a tested polypeptide by directly visualizing and/or quantifying the fragments of IgG which result from cleavage of IgG by a tested polypeptide. Such an assay will typically incubate a sample of IgG with a test polypeptide (or mature IdeS and/or mature IdeZ and/or IdeSORK2.0 as a control) at differing concentrations in a titration series. The products which result from incubation at each concentration are then separated using gel electrophoresis, for example by SDS-PAGE. Whole IgG and the fragments which result from cleavage of IgG can then be identified by size and quantified by the intensity of staining with a suitable dye. The greater the quantity of cleavage fragments, the greater the potency of a tested polypeptide at a given concentration. This type of assay may also enable the identification of test polypeptides that are more effective at cleaving the first or the second heavy chain of an IgG molecule, as the quantities of the different fragments resulting from each cleavage event may also be determined. A polypeptide of the invention may be more effective at cleaving the first chain of an IgG molecule than the second chain.

A polypeptide of the invention is preferably at least as effective at cleaving human IgG1 as IdeSORK2.0.

Suitable methods to assess immunoglobulin protease activity and efficacy are also described in the Examples.

### Structural features of a polypeptide having immunoglobulin protease activity

This section sets out the structural features of a polypeptide having immunoglobulin protease activity, which apply in addition to the functional features outlined in the immediately preceding section. In particular, the structural features apply in addition to the function of immunoglobulin protease activity.

The polypeptide is typically no longer than 600 amino acids in length. The polypeptide may comprise the 557 amino acids of SEQ ID NO: 1 and in addition may be engineered to include one or more additional amino acids upto a total length of 600 amino acids, wherein said additional amino acids are typically to assist with production, isolation or purification. The polypeptide may thus comprise, comprise, consist essentially, or consist of the sequence of SEQ ID NO: 1.

The polypeptide may have a maximum length of 560, 500, 400, or 350 amino acids.

The polypeptide preferably comprises, consists essentially of, or consists of any N terminal fragment of SEQ ID NO: 1, provided it has immunoglobulin protease activity. The polypeptide may comprise, consist essentially of, or consist of the first 150, 160, 170, 180, 190, 200, 210, 220, 230, 240, 250, 260, 270, 280, 290, 300, 301, 302, 303, 304, 305, 306, 307, 308, 309, 310, 311, 312, 313, 314, 315, 316, 317, 318 319, 320, 330, 340, 350, 360, 370, 380, 390 or 400 contiguous amino acids from the N terminus of SEQ ID NO: 1, provided that the polypeptide has immunoglobulin protease activity. The N-terminal fragment preferably comprises at least the first 250 contiguous amino acids from the N terminus of SEQ ID NO: 1, optionally upto the first 320 contiguous amino acids from the N terminus of SEQ ID NO: 1. An exemplary N terminal fragment of SEQ ID NO: 1 is provided as SEQ ID NO: 2. It comprises the first 312 contiguous amino acids from the N terminus of SEQ ID NO: 1.

Any N terminal fragment of SEQ ID NO: 1 may be engineered to include one or more additional amino acids, wherein said additional amino acids are typically to assist with production, isolation or purification. An engineered version of SEQ ID NO: 2 is provided as SEQ ID NO: 3. The immunoglobulin protease activity of the N terminal fragment is preferably at least 50% of the activity against human IgG of the polypeptide consisting of SEQ ID NO: 3, when measured in the same assay, and is more preferably comparable to or superior to the activity of the polypeptide consisting of SEQ ID NO: 3.

Alternatively, the polypeptide of the invention may comprise, consist essentially, or consist of a variant of the amino acid sequence of SEQ ID NO: 1, SEQ ID NO: 2, or any other N-terminal fragment of SEQ ID NO: 1 having immunoglobulin protease activity as defined above. The immunoglobulin protease activity of the variant polypeptide is preferably at least 50% of the activity against human IgG of the polypeptide consisting of SEQ ID NO: 3, when measured in the same assay, and is more preferably comparable to or superior to the activity of the polypeptide consisting of SEQ ID NO: 3.

Said variant may be at least 50% identical to the amino acid sequence of SEQ ID NO: 1, SEQ ID NO: 2, or said N-terminal fragment of SEQ ID NO:1. The variant sequence may be at least 60%, at least 70%, at least 80%, at least, 85%, at least 90%, at least 95%, at least 98% or at least 99% identical to the sequence of SEQ ID NO:1, SEQ ID NO: 2, or said N-terminal fragment of SEQ ID NO:1. The identity level is preferably at least 85% or higher. Identity relative to the sequence of SEQ ID NO: 1, SEQ ID NO: 2, or said N-terminal fragment of SEQ ID NO:1 can be measured over a region of at least 150, 160, 170, 180, 190, 200, 210, 220, 230, 240, 250, 260, 270, 280, 290, 300, 301, 302, 303, 304, 305, 306, 307, 308, 309, 310, 311, 312, 313, 314, 315, 316, 317, 318 319, 320, 330, 340, 350, 360, 370, 380, 390, 400 or more contiguous amino acids of the sequence shown in SEQ ID NO: 1, SEQ ID NO: 2, or said N-terminal fragment of SEQ ID NO:1. More preferably, identity is measured over the full length of SEQ ID NO: 1, SEQ ID NO: 2, or said N-terminal fragment of SEQ ID NO:1. Identity to SEQ ID NO: 1, SEQ ID NO: 2, or said N-terminal fragment of SEQ ID NO:1 may be measured over the length of the variant, provided the variant is of a length which is no more than 50 amino acids longer or shorter than the reference sequence, and is preferably of approximately (or exactly) the same length as the reference sequence. SEQ ID NO: 2 is the most preferred reference sequence. Identity of a variant is most preferably measured over the full length of SEQ ID NO: 2.

Amino acid identity may be calculated using any suitable algorithm. For example the PILEUP and BLAST algorithms can be used to calculate identity or line up sequences (such as identifying equivalent or corresponding sequences (typically on their default settings), for example as described in Altschul S. F. (1993) J Mol Evol 36:290-300; Altschul, S, F et al (1990) J Mol Biol 215:403-10. Software for performing BLAST analyses is publicly available through the National Center for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two polynucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001. Alternatively, the UWGCG Package provides the BESTFIT program which can be used to calculate identity (for example used on its default settings) (Devereux et al (1984) Nucleic Acids Research 12, 387-395).

The sequence of a polypeptide of the invention may comprise a variant of the amino acid sequence of SEQ ID NO: 1, SEQ ID NO: 2, or any other N-terminal fragment of SEQ ID NO: 1 having immunoglobulin protease activity as defined above, in which modifications, such as amino acid additions, deletions or substitutions are made relative to the sequence of SEQ ID NO: 1, SEQ ID NO: 2, or said other N-terminal fragment of SEQ ID NO: 1.

Unless otherwise specified, the modifications are preferably conservative amino acid substitutions. Conservative substitutions replace amino acids with other amino acids of similar chemical structure, similar chemical properties or similar side-chain volume. The amino acids introduced may have similar polarity, hydrophilicity, hydrophobicity, basicity, acidity, neutrality or charge to the amino acids they replace. Alternatively, the conservative substitution may introduce another amino acid that is aromatic or aliphatic in the place of a pre-existing aromatic or aliphatic amino acid. Conservative amino acid changes are well-known in the art and may be selected in accordance with the properties of the 20 main amino acids as defined in Table A1 below. Where amino acids have similar polarity, this can be determined by reference to the hydropathy scale for amino acid side chains in Table A2. A sequence of a polypeptide of the invention may comprise a variant of the amino acid sequence of SEQ ID NO: 1 in which up to 10, 20, 30, 40, 50 or 60 conservative substitutions are made.

**Table A1 - Chemical properties of amino acids**

| | | | |
|---|---|---|---|
| Ala (A) | aliphatic, hydrophobic, neutral | Met (M) | hydrophobic, neutral |
| Cys (C) | polar, hydrophobic, neutral | Asn (N) | polar, hydrophilic, neutral |
| Asp (D) | polar, hydrophilic, charged (-) | Pro (P) | hydrophobic, neutral |
| Glu (E) | polar, hydrophilic, charged (-) | Gln (Q) | polar, hydrophilic, neutral |
| Phe (F) | aromatic, hydrophobic, neutral | Arg (R) | polar, hydrophilic, charged (+) |
| Gly (G) | aliphatic, neutral | Ser (S) | polar, hydrophilic, neutral |
| His (H) | aromatic, polar, hydrophilic, charged (+) | Thr (T) | polar, hydrophilic, neutral |
| Ile (I) | aliphatic, hydrophobic, neutral | Val (V) | aliphatic, hydrophobic, neutral |
| Lys (K) | polar, hydrophilic, charged(+) | Trp (W) | aromatic, hydrophobic, neutral |
| Leu (L) | aliphatic, hydrophobic, neutral | Tyr (Y) | aromatic, polar, hydrophobic |

**Table A2 - Hydropathy scale**

| Side Chain | Hydropathy |
|---|---|
| Ile | 4.5 |
| Val | 4.2 |
| Leu | 3.8 |
| Phe | 2.8 |
| Cys | 2.5 |
| Met | 1.9 |
| Ala | 1.8 |
| Gly | -0.4 |
| Thr | -0.7 |
| Ser | -0.8 |
| Trp | -0.9 |
| Tyr | -1.3 |
| Pro | -1.6 |
| His | -3.2 |
| Glu | -3.5 |
| Gln | -3.5 |
| Asp | -3.5 |
| Asn | -3.5 |
| Lys | -3.9 |
| Arg | -4.5 |

A suitable modification may replace at least one amino acid in the starting sequence with an amino acid having comparable characteristics but which is not one of the 20 L-configuration amino acids appearing in natural eukaryotic proteins. For example, at least one L-configuration amino acid may be replaced with the directly corresponding non-L configuration amino acid, such as a D-configuration amino acid, or with a non-L configuration amino acid which is otherwise a conservative substitution as defined above. The amino acid sequence of a polypeptide of the invention may comprises a variant of the amino acid sequence as described above. However, certain residues are preferably retained within the said variant sequence. For example, numbering the positions starting from the N terminus of SEQ ID NO: 2, the Lys at position 66 is expected to be important for creating the oxyanion hole, the Cys as position 76 and the His at position 226 are predicted to constitute the active site, and the Asp amino acids at positions 248 and 250 are expected to be important for the proper orientation of the active site as well as creating an electrostatic milieu that promotes the hydrolysis. It is preferred that the amino acids in positions corresponding to these positions are not changed, particularly Cys-76 and His-226.

Any polypeptide of the invention which comprises SEQ ID NO:1, SEQ ID NO: 2, or any other N-terminal fragment of SEQ ID NO: 1 having immunoglobulin protease activity as defined above, or any variant of any one of these sequences, may optionally be engineered to include an additional methionine at the N terminus and/or protein purification or other tag at the C terminus. The tag is a sequence which is not naturally expressed in streptococcal bacteria as a contiguous domain of the IdeSORK protein of SEQ ID NO: 1. A preferred protein purification tag is a histidine tag. A histidine tag preferably consists of six histidine residues. The histidine tag may be linked to the C terminus by a linker, which is typically a short sequence of amino acids, such as 3 - 5 amino acids. The linker typically consists predominantly of glycine and serine residues, and may preferably include the sequence GSG. For example GSG and GSGLE are suitable linkers. SEQ ID NO: 3 is an example of an engineered sequence.

In summary therefore, provided herein is:
A polypeptide, optionally an engineered polypeptide, having immunoglobulin protease activity, wherein said polypeptide comprises, consists essentially of, or consists of:
(a) the amino acid sequence of SEQ ID NO: 1;
(b) the amino acid sequence of SEQ ID NO: 2;
(c) an amino acid sequence which is an N terminal fragment of the sequence of SEQ ID NO: 1;
(d) an amino acid sequence which is at least 50% identical to the amino acid sequence of any one of (a), (b) or (c);
optionally wherein said polypeptide is engineered to include an additional methionine at the N terminus and/or a protein purification or other tag at the C terminus, which tag may be joined to the C terminus by a linker.

SEQ ID NOs: 1, 2 and 3 are the sequences of exemplary polypeptides of the invention. The polypeptide may comprise, consist essentially, or consist of the amino acid sequence of any one of SEQ ID NOs: 1, 2 or 3. Exemplary polynucleotide sequences of the invention include SEQ ID NOs: 16 to 19.

### General polypeptide features

A "polypeptide" is used herein in its broadest sense to refer to a compound of two or more subunit amino acids, amino acid analogs, or other peptidomimetics. The term "polypeptide" thus includes short peptide sequences and also longer polypeptides and proteins. The terms "protein", "peptide" and "polypeptide" may be used interchangeably. The term "amino acid" may refer to either natural and/or unnatural or synthetic amino acids, including both D or L optical isomers, and amino acid analogs and peptidomimetics.

A polypeptide may be produced by suitable method, including recombinant or synthetic methods. For example, the polypeptide may be synthesised directly using standard techniques known in the art, such as Fmoc solid phase chemistry, Boc solid phase chemistry or by solution phase peptide synthesis. Alternatively, a polypeptide may be produced by transforming a cell, typically a bacterial cell, with a nucleic acid molecule or vector which encodes said polypeptide. Production of polypeptides by expression in bacterial host cells is described below and is exemplified in the Examples. The invention provides nucleic acid molecules and vectors which encode a polypeptide of the invention. The invention also provides a host cell comprising such a nucleic acid or vector. Exemplary polynucleotide molecules encoding polypeptides disclosed herein are provided as SEQ ID NOs: 16 and 17. Each of these sequences includes at the 5' end a codon for the N terminal methionine (ATG) and, prior to the stop codon (TAA) at the 3' end, codons for a protein purification tag, in this case 6x His tag, which may optionally be excluded. The optional inclusion of an additional methionine and a tag are discussed in more detail elsewhere in this document. SEQ ID NOs: 18 and 19 are expression vector sequences comprising SEQ ID NOs: 16 and 17, respectively.

The terms "nucleic acid molecule" and "polynucleotide" are used interchangeably herein and refer to a polymeric form of nucleotides of any length, either deoxyribonucleotides or ribonucleotides, or analogs thereof. Non-limiting examples of polynucleotides include a gene, a gene fragment, messenger RNA (mRNA), cDNA, recombinant polynucleotides, plasmids, vectors, isolated DNA of any sequence, isolated RNA of any sequence, nucleic acid probes, and primers. A polynucleotide of the invention encodes a polypeptide of the invention and may be provided in isolated or substantially isolated form. By substantially isolated, it is meant that there may be substantial, but not total, isolation of the polypeptide from any surrounding medium. The polynucleotides may be mixed with carriers or diluents which will not interfere with their intended use and still be regarded as substantially isolated. A nucleic acid sequence which "encodes" a selected polypeptide is a nucleic acid molecule which is transcribed (in the case of DNA) and translated (in the case of mRNA) into a polypeptide in vivo when placed under the control of appropriate regulatory sequences, for example in an expression vector. The boundaries of the coding sequence are determined by a start codon at the 5' (amino) terminus and a translation stop codon at the 3' (carboxy) terminus. For the purposes of the invention, such nucleic acid sequences can include, but are not limited to, cDNA from viral, prokaryotic or eukaryotic mRNA, genomic sequences from viral or prokaryotic DNA or RNA, and even synthetic DNA sequences. A transcription termination sequence may be located 3' to the coding sequence.

Polynucleotides can be synthesised according to methods well known in the art, as described by way of example in Sambrook et al (1989, Molecular Cloning - a laboratory manual; Cold Spring Harbor Press). The nucleic acid molecules of the present invention may be provided in the form of an expression cassette which includes control sequences operably linked to the inserted sequence, thus allowing for expression of the polypeptide of the invention in vivo (e.g. in prokaryotic or eukaryotic expression systems). These expression cassettes, in turn, are typically provided within vectors (e.g., plasmids or recombinant viral vectors). Such an expression cassette may be administered directly to a host subject. Alternatively, a vector comprising a polynucleotide of the invention may be administered to a host subject. Preferably the polynucleotide is prepared and/or administered using a genetic vector. A suitable vector may be any vector which is capable of carrying a sufficient amount of genetic information, and allowing expression of a polypeptide of the invention.

The present invention thus includes expression vectors that comprise such polynucleotide sequences. Such expression vectors are routinely constructed in the art of molecular biology and may for example involve the use of plasmid DNA and appropriate initiators, promoters, enhancers and other elements, such as for example polyadenylation signals which may be necessary, and which are positioned in the correct orientation, in order to allow for expression of a peptide of the invention. Other suitable vectors would be apparent to persons skilled in the art. By way of further example in this regard we refer to Sambrook *et al.*

The invention also includes cells that have been modified to express a polypeptide of the invention. Such cells typically include prokaryotic cells such as bacterial cells, for example *E. coli.* Such cells may be cultured using routine methods to produce a polypeptide of the invention.

A polypeptide may be engineered or modified to assist with production, isolation or purification. For example, where a polypeptide of the invention is produced by recombinant expression in a bacterial host cell, the sequence of the polypeptide may include an additional methionine (M) residue at the N terminus to improve expression. As another example, the polypeptide of the invention may be engineered or modified by addition of protein purification tag and the N or C terminus, preferably at the C terminus. The protein purification tag is preferably a moiety which is not naturally expressed in streptococcus bacteria. The protein purification tag is preferably a moiety which is not present in a wildtype polypeptide chain as expressed in streptococcus bacteria. A protein purification tag may be a ligand which is capable of binding directly and specifically to a separation means. Alternatively, the protein purification tag may be one member of a binding pair and the separation means comprises a reagent that includes the other member of the binding pair. Any suitable binding pair can be used.

Where the polypeptide is engineered or modified by addition of one member of a binding pair, the polypeptide is preferably histidine-tagged or biotin-tagged. Typically the amino acid coding sequence of the histidine or biotin tag is included at the gene level and the polypeptide is expressed recombinantly in *E. coli.* The histidine or biotin tag is typically present at either end of the polypeptide, preferably at the C-terminus. It may be joined directly to the polypeptide or joined indirectly by any suitable linker sequence, such as 3, 4 or 5 glycine residues, or a mixture of glycine and serine residues. The histidine tag typically consists of six histidine residues, although it can be longer than this, typically up to 7, 8, 9, 10 or 20 amino acids or shorter, for example 5, 4, 3, 2 or 1 amino acids.

Alternative tags useful for protein purification include Maltose-binding protein (MBP), glutathione S-transferase (GST), thioredoxin (TRX), ubiquitin (Ub), small ubiquitin related modifier (SUMO), solubility-enhancer peptide sequences (SET), and N-utilization substance (NusA). Suitable tags are also discussed in Costa et al; Front Microbiol. 2014; 5: 63, which is incorporated by reference, including in particular each of the tags that are disclosed in Table 1 of Costa *et al.*

The amino acid sequence of a polypeptide may be modified or engineered to include at least one non-naturally occurring amino acid, for example to increase stability. When the polypeptides are produced by synthetic means, such amino acids may be introduced during production. The polypeptides may also be modified following either synthetic or recombinant production. Polypeptides may also be produced using D-amino acids. In such cases the amino acids will be linked in reverse sequence in the C to N orientation. This is conventional in the art for producing such polypeptides. Preferred polypeptides of the invention may be engineered to include at least one such unnatural or synthetic amino acid, or at least one non-L configuration amino acid.

A number of side chain modifications are known in the art and may be made to the side chains of the polypeptides, subject to the polypeptides retaining any further required activity or characteristic as may be specified herein. It will also be understood that polypeptides may be chemically modified, e.g. post-translationally modified. For example, they may be glycosylated, phosphorylated or comprise modified amino acid residues. The polypeptide may be PEGylated.

A polypeptide may be provided in a substantially isolated or purified form. That is, isolated from the majority of the other components present in a cellular extract from a cell in which the polypeptide was expressed. The polypeptide may be mixed with carriers or diluents (as discussed below) which will not interfere with the intended use and still be regarded as substantially isolated. It may also be in a substantially purified form, in which case it will generally comprise at least 90%, e.g. at least 95%, 98% or 99%, of the protein in the preparation. Where a polypeptide is provided in a composition with an additional active component, such as another polypeptide, each said polypeptide will individually be purified to a high level of homogeneity prior to mixing in an appropriate ratio for the intended purpose of each. For example, two polypeptides may be each be purified to at least 90% homogeneity prior to combining in a 1:1 ratio. Exemplary polypeptides that may be included in a mixture with a polypeptide of the invention may include any one or more of: an exoglycosidase; an endoglycosidase (e.g. EndoS, EndoS2, EndoE, including any polypeptide with endoglycosidase activity as disclosed in WO2013/037824); a protease (preferably an immunoglobulin protease with different specificity to the polypeptide of the invention, e.g. a protease as disclosed in WO2017/134274 - such as a polypeptide comprising SEQ ID NO: 3 of that document or a variant thereof as disclosed in that document); or an amidase (e.g. PngaseF).

A polypeptide may be provided in lyophilised form, suitable for reconstitution in aqueous solution prior to use. The lyophilised composition has improved stability enabling longer storage of the polypeptide. A polypeptide is typically substantially purified prior to freeze-drying. A method of preparing a polypeptide in lyophilised form, comprising freeze-drying said polypeptide in a suitable buffer, such as Phosphate-buffered saline (PBS), Tris-buffered saline (TBS), or another Tris-buffer is provided herein. The resulting polypeptide in lyophilised form is also provided. A method of preparing a solution of a polypeptide, comprising providing the polypeptide in lyophilised form and reconstituting with a suitable carrier or diluent, such as water, is also provided.

A polypeptide may be immobilised using methods known in the art, for example as described in Datta S et al., Enzyme immobilization: an overview on techniques and support materials, 3 Biotech, 3(1): 1-9 (2013). For example, the polypeptide may be immobilised by adsorption, covalent binding, affinity immobilization or entrapment. Materials that can be used as supports include but are not limited to for example, natural supports such as agarose, sepharose, collagen, gelatin, cellulose, pectin, sepharose, inorganic materials such as ceramics, silica, glass, activated carbon or charcoal, or synthetic polymers, such as Poly(styrene-divinylbenzene), or latex. Any of these may be provided as a resin or in any other suitable format. The polypeptide may be immobilised on magnetic beads.

### Compositions and formulations comprising polypeptides

In another aspect, the present invention provides compositions comprising a polypeptide of the invention. For example, the invention provides a composition comprising one or more polypeptides of the invention and at least one excipient, preferably a preservative or stabiliser, and optionally also one or more additional carriers, diluents or vehicles. Exemplary classes of excipient and individual examples are included in the following table.

| **Excipient** | **Examples** |
|---|---|
| Salts | Ammonium sulfate, calcium chloride, magnesium sulfate, magnesium chloride, potassium chloride, sodium chloride, sodium gluconate, sodium sulfate, zinc chloride |
| Buffers | Acetate, carbonate, citrate, citrate-phosphate, HEPES, histidine, maleate, phosphate, succinate, tartrate, triethanolamine (Tris) |
| Sugars and polyols | Cyclodextrins, fructose, glucose, glycerol, inositol, lactose, maltose, mannitol, sorbitol, sucrose, trehalose |
| Amino acids | Alanine, arginine, aspartic acid, glycine, lysine, proline |
| Surfactants | Poloxamer 188/407, polysorbate 20/40/80, sodium lauryl sulfate |
| Antioxidants and preservatives | Ascorbic acid, benzyl alocohol, benzoic acid, citric acid, chlorobutanol, *m*-cresol, glutathione, methionine, methylparaben, phenol, propylparaben, sodium sulphite |
| Polymers | Dextran, polyethylene glycol |
| Other | Albumin, dimethyl sulfoxide, EDTA, ehanol, thioglycolic acid |

Typically, the final composition is sterile and pyrogen free.

In a particular embodiment, all of the present excipients are preferably pharmaceutically acceptable, in the sense of being compatible with the other ingredients of the composition and not deleterious to a subject to which the composition is administered. In this case, the composition may be referred to as a pharmaceutical composition.

Formulation of a suitable composition can be carried out using standard pharmaceutical formulation chemistries and methodologies all of which are readily available to the reasonably skilled artisan. For example, the agent can be combined with a preservative, and one or more carriers, excipients or vehicles. Auxiliary substances, such as wetting or emulsifying agents, pH buffering substances, reducing agents and the like, may be present in the excipient or vehicle. Suitable reducing agents include cysteine, thioglycerol, thioreducin, glutathione and the like. Excipients, vehicles and auxiliary substances are generally pharmaceutical agents that do not induce an immune response in the individual receiving the composition, and which may be administered without undue toxicity. Pharmaceutically acceptable excipients include, but are not limited to, liquids such as polyethyleneglycol, hyaluronic acid, glycerol, thioglycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

Such compositions may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. Such compositions may further comprise one or more additional ingredients including, but not limited to, suspending, stabilizing, or dispersing agents. In one embodiment of a composition for parenteral administration, the active ingredient is provided in dry (for e.g., a powder or granules) form for reconstitution with a suitable vehicle (e. g., sterile pyrogen-free water) prior to parenteral administration of the reconstituted composition. The compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono-or di-glycerides.

Other parentally-administrable compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The compositions may be suitable for administration by any suitable route including, for example, intradermal, subcutaneous, percutaneous, intramuscular, intra-arterial, intraperitoneal, intraarticular, intraosseous or other appropriate administration routes. Preferred compositions are suitable for administration by intravenous infusion.

### Methods of use of polypeptides

The polypeptides of the invention are useful in various methods. Thus, provided herein are methods comprising administering a polypeptide of the invention, for example to a sample or a subject. The method may be a method of cleaving an immunoglobulin, and may optionally further comprises the detection or analysis of the cleavage products. The method may be a method treating a disease, the method comprising administering a polypeptide of the invention to a subject.

For example, the present polypeptides may provide useful tools for biotechnology. The polypeptides may be used in a method for the *ex vivo* cleavage of IgG. The IgG may be human, mouse, horse or guinea pig IgG. The IgG may be a recombinant, monoclonal antibody derived from any of these species. The polypeptide may be administered to a sample containing IgG and incubated under conditions which permit immunoglobulin protease activity to occur.

Suitable conditions include incubation for at least 20 minutes, 30 minutes, 40 minutes, 50 minutes, 60 minutes, 70 minutes, 80 minutes, 90 minutes or 120 minutes, 3 hours, 5 hours, 10 hours, or overnight, typically with mixing e.g. end-over-end mixing. A preferred incubation time is around 30 minutes or around 1 hour.

Incubation preferably takes place at room temperature, more preferably at approximately 20°C, 25°C, 30°C, 35°C, 40°C or 45°C, and most preferably at approximately 37°C.

The methods described above may be carried out under any suitable pH. Suitable pH values include, for example, around pH 6.5 to around pH 8.5, preferably around pH 7.0 to around pH 8.5, most preferably at around pH 7.5.

The method may be conducted in any suitable buffer, such as tris buffered saline (TBS) or phosphate buffered saline (PBS). There is preferably no requirement for salts, co-factors or reducing agents, although the presence of salts (e.g. NaCl) at up to 200mM is preferably tolerated by the polypeptide of the invention.

The approximate ratio of the polypeptide of the invention to the protein content of the sample may be 1:1, 2:1, 4:1, 6:1, 10:1, 15:1, 20:1, 1:2, 1:4, or 1:6, 1:10, 1:15, 1:20, 1:40, 1:100, 1:200, 1:400, 1:500, 1:600, 1:700, 1:800, 1:900, 1:1000, 1:1500, 1:2000, or up to 1:2500 (wt:wt). A preferred ratio is between 1:20 and 1:40 (wt:wt).

Specific cleavage can be verified, and the cleavage products isolated using any suitable method, such as those described elsewhere in this document, including in the Examples, or in WO2003051914 and WO2009033670. The method can be used in particular to generate Fc and F(ab')₂ fragments. Fab fragments may then be produced by carrying out a reduction step (for example in 2-mercaptoethanolamine or Cysteamine) on the F(ab')₂ fragments that result from cleavage of IgG with a polypeptide of the invention.

The method may also be used to detect or analyse IgG in a sample, or to remove IgG from a sample. A method for the detection of IgG in a sample typically involves incubating the polypeptide with the sample under conditions which permit binding and cleavage. The presence of IgG can be verified by detection of the specific IgG cleavage products, which may subsequently be analysed.

The polypeptides may also be used in therapy or prophylaxis. In therapeutic applications, polypeptides or compositions are administered to a subject already suffering from a disorder or condition, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as "therapeutically effective amount". In prophylactic applications, polypeptides or compositions are administered to a subject not yet exhibiting symptoms of a disorder or condition, in an amount sufficient to prevent or delay the development of symptoms. Such an amount is defined as a "prophylactically effective amount". The subject may have been identified as being at risk of developing the disease or condition by any suitable means. Thus the invention also provides a polypeptide of the invention for use in the treatment of the human or animal body. Also provided herein is a method of prevention or treatment of disease or condition in a subject, which method comprises administering a polypeptide of the invention to the subject in a prophylactically or therapeutically effective amount. The polypeptide is preferably administered by intravenous infusion, but may be administered by any suitable route including, for example, intradermal, subcutaneous, percutaneous, intramuscular, intra-arterial, intraperitoneal, intraarticular, intraosseous or other appropriate administration routes. The amount of said polypeptide that is administered may be at least about 0.01mg/kg BW, at least about 0.05, 0.1, 0.15, 0.2, 0.2 or 0.25 mg/kg BW, up to at most about 0.5 mg/kg BW, at most about 1.0, 1.5, 2.0, or 2.5 mg/kg BW. Any of the above lower limits may be combined with any of the upper limits to provide a dose range. The amount is preferably between 0.2 and 1.2 mg/kg BW.

Polypeptides of the invention may be particularly useful in a method for the treatment or prevention of a disease or condition mediated by IgG antibodies, which in this context may be described as pathogenic IgG antibodies. Accordingly, the invention provides a polypeptide of the invention for use in the treatment or prevention of a disease or condition, particularly a disease or condition mediated by pathogenic IgG antibodies. The invention also provides a method of treating or preventing a disease or condition, particularly a disease or condition mediated by pathogenic IgG antibodies, comprising administering to an individual a polypeptide of the invention. The method may comprise repeat administration of the said polypeptide. The invention also provides a polypeptide of the invention for use in the manufacture of a medicament for the treatment or prevention of a disease or condition mediated by pathogenic IgG antibodies, particularly a disease or condition mediated by pathogenic IgG antibodies.

Numerous autoimmune diseases are mediated in whole or in part by pathogenic IgG antibodies. Thus, pathogenic antibodies may typically be specific for an antigen which is targeted in an autoimmune disease or other condition mediated wholly or in part by IgG antibodies. Exemplary diseases and conditions mediated by antibodies and the associated antigens are listed in detail in Table D of WO 2016/128559. A polypeptide of the invention may be used in a method to treat any of these diseases or conditions.

Pathogenic antibodies may recognise a tissue or organ transplant received by the subject. Thus the disease treated or prevented by the polypeptide may be antibody mediated transplant rejection. The pathogenic antibodies may recognise another therapeutic agent administered to the subject, such as an antibody, a gene therapy (e.g. a viral or other vector), a replacement for a defective endogenous factor such as an enzyme, a growth or a clotting factor, or a cell therapy. Thus, the disease or condition treated or prevented by the polypeptide may be any antibody response of the subject which reduces the efficacy or benefit to the subject of said therapeutic agent.

Methods of the invention may involve cleavage of a sample containing a known antibody. The antibody is preferably a recombinant monoclonal antibody. The antibody may be Abagovomab, Abciximab,Actoxumab, Adalimumab, Adecatumumab, Afelimomab, Afutuzumab, Alacizumab pegol, ALD518, Alemtuzumab, Alirocumab, Altumomab pentetate, Amatuximab, Anatumomab mafenatox, Anrukinzumab, Apolizumab, Arcitumomab, Aselizumab, Atinumab, Atlizumab (= tocilizumab), Atorolimumab, Bapineuzumab, Basiliximab, Bavituximab, Bectumomab, Belimumab, Benralizumab, Bertilimumab, Besilesomab, Bevacizumab, Bezlotoxumab, Biciromab, Bimagrumab, Bivatuzumab mertansine, Blinatumomab, Blosozumab, Brentuximab vedotin, Briakinumab, Brodalumab, Canakinumab, Cantuzumab mertansine, Cantuzumab ravtansine, Caplacizumab, Capromab pendetide, Carlumab, Catumaxomab, CC49, Cedelizumab, Certolizumab pegol, Cetuximab, Ch.14.18, Citatuzumab bogatox, Cixutumumab, Clazakizumab, Clenoliximab, Clivatuzumab tetraxetan, Conatumumab, Concizumab, Crenezumab, CR6261, Dacetuzumab, Daclizumab, Dalotuzumab, Daratumumab, Demcizumab, Denosumab, Detumomab, Dorlimomab aritox, Drozitumab, Duligotumab, Dupilumab, Dusigitumab, Ecromeximab, Eculizumab, Edobacomab, Edrecolomab, Efalizumab, Efungumab, Elotuzumab Elsilimomab, Enavatuzumab, Enlimomab pegol, Enokizumab, Enoticumab, Ensituximab, Epitumomab cituxetan, Epratuzumab, Erlizumab, Ertumaxomab, Etaracizumab, Etrolizumab, Evolocumab, Exbivirumab, Fanolesomab, Faralimomab Farletuzumab, Fasinumab, FBTA05, Felvizumab, Fezakinumab, Ficlatuzumab, Figitumumab, Flanvotumab, Fontolizumab, Foralumab, Foravirumab, Fresolimumab, Fulranumab, Futuximab, Galiximab,Ganitumab, Gantenerumab, Gavilimomab, Gemtuzumab ozogamicin, Gevokizumab, Girentuximab,Glembatumumab vedotin, Golimumab, Gomiliximab,GS6624, Ibalizumab, Ibritumomab tiuxetan, Icrucumab, Igovomab, Imciromab, Imgatuzumab, Inclacumab, Indatuximab ravtansine, Infliximab, Intetumumab, Inolimomab, Inotuzumab ozogamicin, Ipilimumab, Iratumumab, Itolizumab, Ixekizumab, Keliximab, Labetuzumab, Lampalizumab, Lebrikizumab, Lemalesomab, Lerdelimumab, Lexatumumab, Libivirumab, Ligelizumab, Lintuzumab, Lirilumab, Lodelcizumab, Lorvotuzumab mertansine, Lucatumumab, Lumiliximab, Mapatumumab, Maslimomab, Mavrilimumab, Matuzumab, Mepolizumab, Metelimumab, Milatuzumab, Minretumomab, Mitumomab, Mogamulizumab, Morolimumab, Motavizumab, Moxetumomab pasudotox, Muromonab-CD3, Nacolomab tafenatox, Namilumab, Naptumomab estafenatox, Narnatumab, Natalizumab, Nebacumab, Necitumumab, Nerelimomab, Nesvacumab, Nimotuzumab, Nivolumab, Nofetumomab merpentan, Obinutuzumab, Ocaratuzumab, Ocrelizumab, Odulimomab, Ofatumumab, Olaratumab, Olokizumab, Omalizumab, Onartuzumab, Oportuzumab monatox, Oregovomab, Orticumab, Otelixizumab, Oxelumab, Ozanezumab, Ozoralizumab, Pagibaximab, Palivizumab, Panitumumab, Panobacumab, Parsatuzumab, Pascolizumab, Pateclizumab, Patritumab, Pemtumomab, Perakizumab, Pertuzumab, Pexelizumab, Pidilizumab, Pinatuzumab vedotin, Pintumomab, Placulumab, Polatuzumab vedotin, Ponezumab, Priliximab, Pritoxaximab, Pritumumab, PRO 140, Quilizumab, Racotumomab, Radretumab, Rafivirumab, Ramucirumab, Ranibizumab,Raxibacumab, Regavirumab, Reslizumab, Rilotumumab, Rituximab, Robatumumab, Roledumab, Romosozumab, Rontalizumab, Rovelizumab, Ruplizumab, Samalizumab, Sarilumab, Satumomab pendetide, Secukinumab, Seribantumab, Setoxaximab, Sevirumab, Sibrotuzumab, Sifalimumab, Siltuximab, Simtuzumab, Siplizumab, Sirukumab, Solanezumab, Solitomab, Sonepcizumab, Sontuzumab, Stamulumab, Sulesomab, Suvizumab, Tabalumab, Tacatuzumab tetraxetan, Tadocizumab, Talizumab, Tanezumab, Taplitumomab paptox, Tefibazumab, Telimomab aritox, Tenatumomab, Teneliximab, Teplizumab, Teprotumumab, TGN1412, Ticilimumab (= tremelimumab), Tildrakizumab, Tigatuzumab, TNX-650, Tocilizumab (= atlizumab), Toralizumab, Tositumomab, Tralokinumab, Trastuzumab, TRBS07, Tregalizumab, Tremelimumab Tucotuzumab celmoleukin, Tuvirumab, Ublituximab, Urelumab, Urtoxazumab, Ustekinumab, Vapaliximab, Vatelizumab, Vedolizumab, Veltuzumab,Vepalimomab Vesencumab, Visilizumab, Volociximab, Vorsetuzumab mafodotin, Votumumab, Zalutumumab, Zanolimumab, Zatuximab, Ziralimumab or Zolimomab aritox.

The following Examples illustrate the invention.

### Example 1

Unless indicated otherwise, the methods used are standard biochemistry and molecular biology techniques. Examples of suitable methodology textbooks include Sambrook et al., Molecular Cloning, A Laboratory Manual (1989) and Ausubel et al., Current Protocols in Molecular Biology (1995), John Wiley and Sons, Inc.

### Analysis of new protease

Several years ago, a study was performed upon which many different rodents (moles and voles) were caught, and streptococcal species thereof were isolated and partly characterized. One of these isolates, tentatively called Streptococcus krösus, was sequenced and was found to have several glycosidases and proteases. One gene in particular had a low degree of homology to IdeS. This gene, referred to herein as IdeSORK, is characterised below.

### Production and purification

The full length protein of SEQ ID NO: 1 (IdeSORK) consistently expressed poorly and to a low purity (Fig 1). However, a truncated version lacking a ca 20 kDa long C-terminal Domain of SEQ ID NO: 1, engineered with N terminal M and C terminal His tag (IdeSORK2.0; SEQ ID NO: 3) behaves well, and can be expressed in E. coli BL21(DE3) STAR cells as a soluble protein with high purity and rather high expression. The expression and purification follows standard protocols, culturing cells at 37°C, inducing using 1 mM IPTG, and harvesting cells after 5 hours of induction. When purifying (GraviTRAP) material after sonication, we often get a yield around 5 mg/g. Modifying the temperature to 30°C during induction can significantly improve the production, reaching ca 9 mg/g (data not shown).

### Activity

### Specificity

IdeSORK2.0 shows no signs of general protease activity towards casein/gelatin (Fig 2), while hydrolyzing all tested human IgGs and IgG Fc fusion proteins (Fig 3). IdeSORK2.0 is not active towards IgA, indicating that it is not the general immunoglobulin structure that dictates activity, but rather an even higher specificity (Fig 3). The enzyme has a slight preference for IgG1 over IgG2, similar to IdeS, and can also hydrolyze monoclonal IgG4. For mouse IgG, the protease is active against mIgG2a, but not mIgG1 or mIgG2b. Alterations of the hinge region of human antibodies (e.g. LALA-sequence) did not affect the activity of IdeSORK2.0. Incubation with polyclonal IgG (e.g. IVIG) resulted in near complete hydrolysis indicating that the enzyme is not limited to specific monoclonal antibodies but can hydrolyze all human IgG. A further examination towards more animal IgG (porcine, horse, dog, rat, guinea pig, sheep, cow) only showed activity against horse and guinea pig IgG (Fig 11).

### Protease Inhibitor sensitivity

IdeS is characterized as a cysteine protease, and is as such not affected by most protease inhibitors other than E64. The enzymatic mechanism of the new protease identified here has not been fully characterized, but IdeSORK2.0 is highly sensitive for many protease inhibitors, including known serine and cysteine protease inhibitors (Fig 4). The enzyme does however not depend on cations, and is unaffected by presence of EDTA (Fig 4).

### Site of hydrolysis

Several substrates (IgG1, IgG2, fusion proteins, LALA-mutated IgG) were subjected to hydrolysis by IdeSORK2.0 and the site of hydrolysis identified through LC-MS. As shown in Fig 5, only a single hydrolytic site was detected, being identical in all sequences, and corresponding to the human IgG1 hinge/CH2 region (see Table 1 above and also the table below). This is the same site that is recognized by IdeS. The site of hydrolysis has been confirmed for etanercept, trastuzumab, cetuximab, as well as two non-commercial monoclonal antibodies.

| Name | Description | Digestion site |
|---|---|---|
| Non-commercial mAb | Human IgG1 | CPPCPAPELLG / GPSVFLFPPKP |
| Trastuzumab | Human IgG1 | CPPCPAPELLG / GPSVFLFPPKP |
| Non-commercial mAb | Human IgG1 LALA modified | CPPCPAPEAAG / GPSVFLFPPKP |
| Cetuximab | Chimeric human /mouse IgG1 | CPPCPAPELLG / GPSVFLFPPKP |
| Etanercept | Dimeric human IgG1 -TNFR fusion protein | CPPCPAPELLG / GPSVFLFPPKP |

### Mechanism of action

IdeS is known to first hydrolyze one chain of the antibody, and upon hydrolysis thereof, have lower affinity to the second chain. As such many antibodies may be only partly hydrolyzed at the start of the reaction. IdeSORK2.0 also generates fragments with single chain cleavage, however a large proportion of these are quickly processed to full cleavage (Fig 6). The affinity for the second chain has not been determined, but it is clear that activity is mediated one chain at a time.

### Optimal conditions

The optimal conditions for IdeSORK2.0 were assessed using a Box-Behnken experimental design to assess multiple factors and their inter-dependence. We assayed the importance of pH, NaCl and temperature for the protease, and the results are summarized in Fig 7. IdeSORK2.0 is highly active in pH 7.0-8.5 and tolerates NaCl up to 200 mM without a significant drop in activity. The activity is improved when incubated at 37°C as compared to room temperature. The enzymatic reaction is mainly driven by the substrate concentration (high concentration favours the reaction), while the enzyme concentration is secondary. This can also be seen as a low percentage IgG not being readily hydrolyzed even after a prolonged incubation (Fig 9). In summary: pH optimum (range): 7.5 (6.5-8.5; >90% activity), NaCl optimum (range): 0 mM (0-200 mM; >90% activity), no requirement for co-factors or reducing agents, high specificity for IgG.

### Time-dose assays

To investigate the impact of substrate and enzyme concentration, as well as time needed to complete (>95% hydrolysis) a reaction, a new Box-Behnken assay was designed (Fig 8). The data was supported by traditional assays, with similar results (Fig 9). IdeSORK2.0 quite rapidly hydrolyzes the vast majority of the material, but needs a longer time to finish hydrolysis of the last percentages of intact IgG, similar to other immunoglobulin proteases. A ratio of 1:40 hydrolyzes >95% of IgG in 30-60 minutes.

### Comparison to other IgG proteases

To further look into the activity of IdeSORK2.0, its activity was compared with IdeS and IdeZ against both IgG1 and IgG2. All enzymes were able to hydrolyze the antibodies in seemingly an identical fashion, though slightly different efficiently (Fig 10).

### Sequence comparison to other IgG proteases

Clustal W alignment was performed for the sequences of IdeSORK, IdeSORK2.0, IdeS and IdeZ (not shown). Although comparable in length to IdeS, IdeSORK2.0 has only around 33% identity, 67% similarity to IdeS in the aligned sequence, which is significantly less than the similarity between IdeS and IdeZ. IdeS and IdeZ have 66% identity, 88% similarity with each other, and cluster together in a phylogenetic tree. Both IdeSORK and the truncated form IdeSORK2.0 are related to each other, but only distantly related to IdeS and IdeZ.

### Materials & Methods

### Expression & Purification of recombinant protein

The vector (20 ng) was transformed into chemically competent E. coli BL21 DE3 STAR cells according to manufacturer's instructions (Invitrogen). Vector sequences are provided as SEQ ID NO: 18 for IdeSORK1.0 and SEQ ID NO: 19 for IdeSORK2.0. The vector used was pet21a+. An overnight culture of bacteria grown in LB supplemented with 100 µg/mL ampicillin, at 37°C, 200 rpm is used to inoculate fresh LB (supplemented with 100 µg/mL ampicillin) at a ratio of 1:40. The incubation continues until OD600 reaches ca 0.6-0.8 upon which the temperature is reduced to 30°C and protein expression is induced by addition of 1 mM IPTG. Expression continues for 5 hours until the cells are collected and stored at -20°C until further processing.

Frozen cells were thawed, and resuspended in binding buffer (5 mL/g cells; 20 mM sodium phosphate pH 7.4, 500 mM NaCl, 20 mM imidazole) before lysis through sonication. Insoluble cell debris was removed by centrifugation (11000 g, 20 min, 4°C), and the lysate purified using a pre-equilibrated His GraviTrap before being buffer exchanged to TBS on a PD10 column. All material was analyzed on SDS-PAGE and Nanodrop for determination of purity and quality of the product.

### Specificity determination using LC-MS

Subunit fragments were denatured and reduced followed by reversed phase LC-MS analysis. Separation was achieved using a BioResolve RP mAb Polyphenol, 450 Å, 2.7 µm 2.1 x 100 mm, Waters (0.1% formic acid in water and 0.1% formic acid in 95% acetonitrile as mobile phases) and run on an Agilent 1260/1290 UHPLC system coupled to a Bruker Impact II QTOF mass spectrometer. The obtained data was evaluated in Bruker Compass DataAnalysis software using the MaxEnt algorithm for deconvolution and SNAP algorithm for determination of the monoisotopic molecular weights.

### General protease activity determination

General protease activity was determined using the EnzChek Protease Assay kit, according to manufacturer's instructions. As a positive control the general protease FabULOUS (SpeB) was used, and as a negative control the IgG-specific protease FabRICATOR (IdeS).

### Protease inhibitor assay

IgG1 was pre-incubated with a set of protease inhibitors (G-Biosciences) before addition of IdeSork2.0 (of SEQ ID NO: 3) (1:5), and the mixture was allowed to incubate overnight at 37°C before analysis on SDS-PAGE.

### Immunoglobulin specificity

Various antibodies and antibody based therapeutics were incubated with IdeSork2.0 (of SEQ ID NO: 3) (1:5 ratio) overnight at 37°C in TBS before being analyzed on SDS-PAGE. When comparing the activity of IdeSork2.0 (of SEQ ID NO: 3) with IdeZ and IdeS, a 1:40 ratio of enzymes were used over an incubation period of 4 hours at 37°C.

### Activity assays

To better understand the optimal conditions needed for the enzyme to exert its activity, a Box-Behnken design was employed for the experiment. Addition of NaCl (0-400 mM), pH (5.5-8.5), [enzyme], [substrate], time, and temperature were parameters investigated. Unless otherwise noted, all experiments were conducted in TBS at an enzyme:substrate ratio of 1:20, for 1 hour at 37°C.

### SEQUENCES

SEQ ID NO: 1 (IdeSORK). Full length sequence of protease isolated from
Streptococcus krösus.
LSKRKLLKKIEKKDTSSVLTQKKQTKTLWADGVQVDDKDFKPSTENFGTNYLAAEYGI
GKGYYDINKKFDGTDDDLCSGVVAANQLHWWLDRNKDYIEKYRQQSKDNGVTIGNT
DIFELNKLHDEDQSNFFDFIKKSFGNKFLQPERLLNMYINGYGYLTSQDKAKTSQPSP
SKLNFFQKVFKNNLLTDKTPINDIDEFSSQTKNALQNHKVLAVSFASIKNRGLGHVVTV
WGADFDENGKVVALYVTDSDDRSKNIGNAKLGMKKLRIEVSAQDSSTIKLTGFEDKN
SGGSLRHLYSLSTGEQIWKKYFEETEKERIRLEEEADKAKLEQDRIQKEAEEKLALEKA
EKERIRLEEEADKAKLEQDRIQKEAEEKLALEKAEKERIRLEEEADKAKLEQDRIQKEAEE
KLALEKAEKERIRLEEEADKAKLEQDRIQKEAEEKLALEKAEKERIRLEEEAAKAKLEQEK
QIATAPQPDKKQENTTSEQEKPAPTELPPLVNKADETETPRETAPDQTPSATNTFRKIL
PKMNAVSQFFSQLMGTIQIVFAFILKIFK

SEQ ID NO: 2. N terminal fragment of IdeSORK having immunoglobulin protease
activity.
SKRKLLKKIEKKDTSSVLTQKKQTKTLWADGVQVDDKDFKPSTENFGTNYLAAEYGI
GKGYYDINKKFDGTDDDLCSGVVAANQLHWWLDRNKDYIEKYRQQSKDNGVTIGNT
DIFELNKLHDEDQSNFFDFIKKSFGNKFLQPERLLNMYINGYGYLTSQDKAKTSQPSP
SKLNFFQKVFKNNLLTDKTPINDIDEFSSQTKNALQNHKVLAVSFASIKNRGLGHVVTV
WGADFDENGKVVALYVTDSDDRSKNIGNAKLGMKKLRIEVSAQDSSTIKLTGFEDKN
SGGSLRHLYSLSTGEQIWKKYFEET

SEQ ID NO: 3 (IdeSORK2.0). Protein of SEQ ID NO: 2 engineered to include
an additional N terminal methionine and a C-terminal protein purification
tag - His6.
MSKRKLLKKIEKKDTSSVLTQKKQTKTLWADGVQVDDKDFKPSTENFGTNYLAAEYGI
GKGYYDINKKFDGTDDDLCSGVVAANQLHWWLDRNKDYIEKYRQQSKDNGVTIGNT
DIFELNKLHDEDQSNFFDFIKKSFGNKFLQPERLLNMYINGYGYLTSQDKAKTSQPSP
SKLNFFQKVFKNNLLTDKTPINDIDEFSSQTKNALQNHKVLAVSFASIKNRGLGHVVTV
WGADFDENGKVVALYVTDSDDRSKNIGNAKLGMKKLRIEVSAQDSSTIKLTGFEDKN
SGGSLRHLYSLSTGEQIWKKYFEETHHHHHH

SEQ ID NOS: 4 TO 10 ARE THE SEQUENCES OF THE HINGE/CH2 REGIONS OF VARIOUS HUMAN AND MOUSE IGG SUBCLASSES

| Subclass | Hinge/CH2 sequence | SEQ ID | Subclass | Hinge/CH2 sequence | SEQ ID |
|---|---|---|---|---|---|
| Human | | | Mouse | | |
| IgG1 | CPPCPAPELL**GG**PSVF | 4 | IgG1 | PCICTVPEVSSVF | 10 |
| IgG2 | CPPCPAPPV**AG**PSVF | 5 | IgG2a | CPPCAAPNLL**GG**PSVF | 8 |
| IgG3 | CPRCPAPELL**GG**PSVF | 6 | IgG2b | CHKCPAPNLEGGPSVF | 11 |
| IgG4 | AHHAQAPEFL**GG**PSVF | 7 | IgG3 | GSSCPAGNIL**GG**PSVF | 9 |

SEQ ID NO: 12. Full length IdeS. Also disclosed as NCBI Reference sequence
no. WP_010922160.1.
MRKRCYSTSAAVLAAVTLFVLSVDRGVIADSFSANQEIRYSEVTPYHVTSVWTKGVTPPANFTQGEDVFHAPYVA
NQGWYDITKTFNGKDDLLCGAATAGNMLHWWFDQNKDQIKRYLEEHPEKQKINFNGEQMFDVKEAIDTKNHQLDS

KLFEYFKEKAFPYLSTKHLGVFPDHVIDMFINGYRLSLTNHGPTPVKEGSKDPRGGIFDAVFTRGDQSKLLTSRH
DFKEKNLKEISDLIKKELTEGKALGLSHTYANVRINHVINLWGADFDSNGNLKAIYVTDSDSNASIGMKKYFVGV
NSAGKVAISAKEIKEDNIGAQVLGLFTLSTGQDSWNQTN

SEQ ID NO: 13. Mature IdeS. Also disclosed as Genbank accession no.
ADF13949.1.
DSFSANQEIRYSEVTPYHVTSVWTKGVTPPANFTQGEDVFHAPYVANQGWYDITKTFNGKDDLLCGAATA
GNMLHWWFDQNKDQIKRYLEEHPEKQKINFNGEQMFDVKEAIDTKNHQLDSKLFEYFKEKAFPYLSTKHL
GVFPDHVIDMFINGYRLSLTNHGPTPVKEGSKDPRGGIFDAVFTRGDQSKLLTSRHDFKEKNLKEISDLI
KKELTEGKALGLSHTYANVRINHVINLWGADFDSNGNLKAIYVTDSDSNASIGMKKYFVGVNSAGKVAIS
AKEIKEDNIGAQVLGLFTLSTGQDSWNQTN

SEQ ID NO: 14. Full length IdeZ. Also disclosed as NCBI Reference sequence
no. WP_014622780.1.
MKTIAYPNKPHSLSAGLLTAIAIFSLASSNITYADDYQRNATEAYAKEVPHQITSVWTKGVTPLTPEQFRYNNED
VIHAPYLAHQGWYDITKAFDGKDNLLCGAATAGNMLHWWFDQNKTEIEAYLSKHPEKQKIIFNNQELFDLKAAID
TKDSQTNSQLFNYFRDKAFPNLSARQLGVMPDLVLDMFINGYYLNVFKTQSTDVNRPYQDKDKRGGIFDAVFTRG
DQTTLLTARHDLKNKGLNDISTIIKQELTEGRALALSHTYANVSISHVINLWGADFNAEGNLEAIYVTDSDANAS
IGMKKYFVGINAHGHVAISAKKIEGENIGAQVLGLFTLSSGKDIWQKLS

SEQ ID NO: 15. Mature IdeZ.
DDYQRNATEAYAKEVPHQITSVWTKGVTPLTPEQFRYNNEDVIHAPYLAHQGWYDITKAFDGKDNLLCGAATAGN
MLHWWFDQNKTEIEAYLSKHPEKQKIIFNNQELFDLKAAIDTKDSQTNSQLFNYFRDKAFPNLSARQLGVMPDLV
LDMFINGYYLNVFKTQSTDVNRPYQDKDKRGGIFDAVFTRGDQTTLLTARHDLKNKGLNDISTIIKQELTEGRAL
ALSHTYANVSISHVINLWGADFNAEGNLEAIYVTDSDANASIGMKKYFVGINAHGHVAISAKKIEGENIGAQVLG
LFTLSSGKDIWQKLS

SEQ ID NO: 16 is an exemplary nucleotide sequence encoding the polypeptide
of SEQ ID NO: 1 engineered for expression with an N terminal histidine and
a C terminal His6.
ATGAGCAAGCGTAAACTGCTGAAGAAAATCGAGAAGAAAGACACCAGCAGCGTGCTGACCCAGAAGAAACAAACCAAGACCCTGTGGGCGGATG
GCGTGCAGGTTGACGATAAGGACTTCAAACCGAGCACCGAAAACTTTGGTACCAACTACCTGGCGGCGGAGTATGGTATCGGCAAGGGTTACTA
CGATATCAACAAGAAGTTCGACGGCACCGACGATGACCTGTGCAGCGGTGTGGTTGCGGCGAACCAGCTGCACTGGTGGCTGGATCGTAACAAG
GACTACATTGAAAAATATCGTCAGCAAAGCAAGGATAACGGCGTGACCATCGGTAACACCGACATTTTCGAACTGAACAAACTGCACGATGAGG
ACCAGAGCAACTTCTTTGATTTCATCAAGAAAAGCTTCGGCAACAAGTTTCTGCAACCGGAGCGTCTGCTGAACATGTACATTAACGGCTACGG
TTATCTGACCAGCCAGGATAAGGCGAAAACCAGCCAACCGAGCCCGAGCAAGCTGAACTTCTTTCAGAAGGTTTTCAAAAACAACCTGCTGACC
GACAAAACCCCGATCAACGATATTGACGAATTTAGCAGCCAGACCAAGAACGCGCTGCAAAACCACAAAGTGCTGGCGGTTAGCTTCGCGAGCA
TCAAGAACCGTGGCCTGGGTCACGTGGTTACCGTGTGGGGCGCGGATTTTGACGAGAACGGTAAAGTGGTTGCGCTGTATGTTACCGACAGCGA
TGACCGTAGCAAGAACATTGGCAACGCGAAACTGGGTATGAAGAAACTGCGTATCGAAGTGAGCGCGCAGGATAGCAGCACCATTAAGCTGACC
GGCTTCGAGGACAAAAACAGCGGTGGCAGCCTGCGTCACCTGTACAGCCTGAGCACCGGTGAACAAATCTGGAAGAAGTACTTCGAGGAAACCG
AAAAGGAGCGTATTCGTCTGGAGGAAGAGGCGGATAAGGCGAAACTGGAACAGGACCGTATCCAAAAAGAGGCGGAAGAGAAACTGGCGCTGGA
AAAGGCGGAAAAAGAGCGTATCCGTCTGGAAGAGGAAGCGGACAAAGCGAAGCTGGAACAAGACCGTATTCAAAAAGAGGCGGAGGAAAAACTG
GCGCTGGAGAAAGCGGAGAAGGAACGTATCCGCCTGGAGGAAGAGGCGGACAAAGCGAAACTGGAACAAGATCGCATTCAGAAAGAGGCGGAGG
AGAAGCTGGCGCTGGAAAAAGCGGAGAAGGAGCGCATTCGCCTGGAAGAGGAAGCGGATAAAGCGAAGCTGGAACAGGATCGCATCCAGAAAGA
GGCGGAAGAGAAGCTGGCGCTGGAGAAGGCGGAGAAGGAGCGTATCCGACTGGAGGAAGAGGCGGCGAAAGCTAAACTGGAACAAGAAAAACAA
ATTGCGACCGCGCCGCAGCCGGACAAGAAACAAGAAAACACCACCAGCGAACAGGAGAAACCGGCGCCGACCGAGCTGCCGCCGCTGGTTAACA
AAGCGGATGAAACCGAGACCCCGCGTGAGACCGCGCCGGACCAAACCCCGAGCGCGACCAACACCTTCCGTAAGATCCTGCCGAAAATGAACGC
TGTGAGCCAGTTCTTTAGCCAACTGATGGGTACCATCCAGATTGTTTTCGCGTTTATCCTGAAGATTTTTAAAGGCAGCGGTCATCACCACCAC
CACCACTAA

SEQ ID NO: 17 is an exemplary nucleotide sequence encoding the polypeptide
of SEQ ID NO: 2 engineered for expression with an N terminal histidine and
a C terminal His6. SEQ ID NO: 17 encodes SEQ ID NO: 3.
ATGAGCAAGCGTAAACTGCTGAAGAAAATCGAGAAGAAAGACACCAGCAGCGTGCTGACCCAGAAGAAACAAACCAAGACCCTGTGGGCGGATG
GCGTGCAGGTTGACGATAAGGACTTCAAACCGAGCACCGAAAACTTTGGTACCAACTACCTGGCGGCGGAGTATGGTATCGGCAAGGGTTACTA
CGATATCAACAAGAAGTTCGACGGCACCGACGATGACCTGTGCAGCGGTGTGGTTGCGGCGAACCAGCTGCACTGGTGGCTGGATCGTAACAAG
GACTACATTGAAAAATATCGTCAGCAAAGCAAGGATAACGGCGTGACCATCGGTAACACCGACATTTTCGAACTGAACAAACTGCACGATGAGG
ACCAGAGCAACTTCTTTGATTTCATCAAGAAAAGCTTCGGCAACAAGTTTCTGCAACCGGAGCGTCTGCTGAACATGTACATTAACGGCTACGG
TTATCTGACCAGCCAGGATAAGGCGAAAACCAGCCAACCGAGCCCGAGCAAGCTGAACTTCTTTCAGAAGGTTTTCAAAAACAACCTGCTGACC
GACAAAACCCCGATCAACGATATTGACGAATTTAGCAGCCAGACCAAGAACGCGCTGCAAAACCACAAAGTGCTGGCGGTTAGCTTCGCGAGCA
TCAAGAACCGTGGCCTGGGTCACGTGGTTACCGTGTGGGGCGCGGATTTTGACGAGAACGGTAAAGTGGTTGCGCTGTATGTTACCGACAGCGA
TGACCGTAGCAAGAACATTGGCAACGCGAAACTGGGTATGAAGAAACTGCGTATCGAAGTGAGCGCGCAGGATAGCAGCACCATTAAGCTGACC
GGCTTCGAGGACAAAAACAGCGGTGGCAGCCTGCGTCACCTGTACAGCCTGCATCACCACCACCACCACTAA

SEQ ID NO: 18 is an exemplary expression vector sequence comprising the sequence of SEQ ID NO : 16. The vector is pET21a+. Gene is shaded grey.

TGGCGAATGGGACGCGCCCTGTAGCGGCGCATTAAGCGCGGCGGGTGTGGTGGTTACGCGCAGCGTGACCGCTACACTTGCCAGCGCCCTAGCG
CCCGCTCCTTTCGCTTTCTTCCCTTCCTTTCTCGCCACGTTCGCCGGCTTTCCCCGTCAAGCTCTAAATCGGGGGCTCCCTTTAGGGTTCCGAT
TTAGTGCTTTACGGCACCTCGACCCCAAAAAACTTGATTAGGGTGATGGTTCACGTAGTGGGCCATCGCCCTGATAGACGGTTTTTCGCCCTTT
GACGTTGGAGTCCACGTTCTTTAATAGTGGACTCTTGTTCCAAACTGGAACAACACTCAACCCTATCTCGGTCTATTCTTTTGATTTATAAGGG
ATTTTGCCGATTTCGGCCTATTGGTTAAAAAATGAGCTGATTTAACAAAAATTTAACGCGAATTTTAACAAAATATTAACGTTTACAATTTCAG
GTGGCACTTTTCGGGGAAATGTGCGCGGAACCCCTATTTGTTTATTTTTCTAAATACATTCAAATATGTATCCGCTCATGAGACAATAACCCTG
ATAAATGCTTCAATAATATTGAAAAAGGAAGAGTATGAGTATTCAACATTTCCGTGTCGCCCTTATTCCCTTTTTTGCGGCATTTTGCCTTCCT
GTTTTTGCTCACCCAGAAACGCTGGTGAAAGTAAAAGATGCTGAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACTTTTAAAGTTCTGCTATGTGGCGCGGTATTATCCCGTATTGA
CGCCGGGCAAGAGCAACTCGGTCGCCGCATACACTATTCTCAGAATGACTTGGTTGAGTACTCACCAGTCACAGAAAAGCATCTTACGGATGGC
ATGACAGTAAGAGAATTATGCAGTGCTGCCATAACCATGAGTGATAACACTGCGGCCAACTTACTTCTGACAACGATCGGAGGACCGAAGGAGC
TAACCGCTTTTTTGCACAACATGGGGGATCATGTAACTCGCCTTGATCGTTGGGAACCGGAGCTGAATGAAGCCATACCAAACGACGAGCGTGA
CACCACGATGCCTGCAGCAATGGCAACAACGTTGCGCAAACTATTAACTGGCGAACTACTTACTCTAGCTTCCCGGCAACAATTAATAGACTGG
ATGGAGGCGGATAAAGTTGCAGGACCACTTCTGCGCTCGGCCCTTCCGGCTGGCTGGTTTATTGCTGATAAATCTGGAGCCGGTGAGCGTGGGT
CTCGCGGTATCATTGCAGCACTGGGGCCAGATGGTAAGCCCTCCCGTATCGTAGTTATCTACACGACGGGGAGTCAGGCAACTATGGATGAACG
AAATAGACAGATCGCTGAGATAGGTGCCTCACTGATTAAGCATTGGTAACTGTCAGACCAAGTTTACTCATATATACTTTAGATTGATTTAAAA
CTTCATTTTTAATTTAAAAGGATCTAGGTGAAGATCCTTTTTGATAATCTCATGACCAAAATCCCTTAACGTGAGTTTTCGTTCCACTGAGCGT
CAGACCCCGTAGAAAAGATCAAAGGATCTTCTTGAGATCCTTTTTTTCTGCGCGTAATCTGCTGCTTGCAAACAAAAAAACCACCGCTACCAGC
GGTGGTTTGTTTGCCGGATCAAGAGCTACCAACTCTTTTTCCGAAGGTAACTGGCTTCAGCAGAGCGCAGATACCAAATACTGTCCTTCTAGTG
TAGCCGTAGTTAGGCCACCACTTCAAGAACTCTGTAGCACCGCCTACATACCTCGCTCTGCTAATCCTGTTACCAGTGGCTGCTGCCAGTGGCG
ATAAGTCGTGTCTTACCGGGTTGGACTCAAGACGATAGTTACCGGATAAGGCGCAGCGGTCGGGCTGAACGGGGGGTTCGTGCACACAGCCCAG
CTTGGAGCGAACGACCTACACCGAACTGAGATACCTACAGCGTGAGCTATGAGAAAGCGCCACGCTTCCCGAAGGGAGAAAGGCGGACAGGTAT
CCGGTAAGCGGCAGGGTCGGAACAGGAGAGCGCACGAGGGAGCTTCCAGGGGGAAACGCCTGGTATCTTTATAGTCCTGTCGGGTTTCGCCACC
TCTGACTTGAGCGTCGATTTTTGTGATGCTCGTCAGGGGGGCGGAGCCTATGGAAAAACGCCAGCAACGCGGCCTTTTTACGGTTCCTGGCCTT
TTGCTGGCCTTTTGCTCACATGTTCTTTCCTGCGTTATCCCCTGATTCTGTGGATAACCGTATTACCGCCTTTGAGTGAGCTGATACCGCTCGC
CGCAGCCGAACGACCGAGCGCAGCGAGTCAGTGAGCGAGGAAGCGGAAGAGCGCCTGATGCGGTATTTTCTCCTTACGCATCTGTGCGGTATTT
CACACCGCATATATGGTGCACTCTCAGTACAATCTGCTCTGATGCCGCATAGTTAAGCCAGTATACACTCCGCTATCGCTACGTGACTGGGTCA
TGGCTGCGCCCCGACACCCGCCAACACCCGCTGACGCGCCCTGACGGGCTTGTCTGCTCCCGGCATCCGCTTACAGACAAGCTGTGACCGTCTC
CGGGAGCTGCATGTGTCAGAGGTTTTCACCGTCATCACCGAAACGCGCGAGGCAGCTGCGGTAAAGCTCATCAGCGTGGTCGTGAAGCGATTCA
CAGATGTCTGCCTGTTCATCCGCGTCCAGCTCGTTGAGTTTCTCCAGAAGCGTTAATGTCTGGCTTCTGATAAAGCGGGCCATGTTAAGGGCGG
TTTTTTCCTGTTTGGTCACTGATGCCTCCGTGTAAGGGGGATTTCTGTTCATGGGGGTAATGATACCGATGAAACGAGAGAGGATGCTCACGAT
ACGGGTTACTGATGATGAACATGCCCGGTTACTGGAACGTTGTGAGGGTAAACAACTGGCGGTATGGATGCGGCGGGACCAGAGAAAAATCACT
CAGGGTCAATGCCAGCGCTTCGTTAATACAGATGTAGGTGTTCCACAGGGTAGCCAGCAGCATCCTGCGATGCAGATCCGGAACATAATGGTGC
AGGGCGCTGACTTCCGCGTTTCCAGACTTTACGAAACACGGAAACCGAAGACCATTCATGTTGTTGCTCAGGTCGCAGACGTTTTGCAGCAGCA
GTCGCTTCACGTTCGCTCGCGTATCGGTGATTCATTCTGCTAACCAGTAAGGCAACCCCGCCAGCCTAGCCGGGTCCTCAACGACAGGAGCACG
ATCATGCGCACCCGTGGGGCCGCCATGCCGGCGATAATGGCCTGCTTCTCGCCGAAACGTTTGGTGGCGGGACCAGTGACGAAGGCTTGAGCGA
GGGCGTGCAAGATTCCGAATACCGCAAGCGACAGGCCGATCATCGTCGCGCTCCAGCGAAAGCGGTCCTCGCCGAAAATGACCCAGAGCGCTGC
CGGCACCTGTCCTACGAGTTGCATGATAAAGAAGACAGTCATAAGTGCGGCGACGATAGTCATGCCCCGCGCCCACCGGAAGGAGCTGACTGGG
TTGAAGGCTCTCAAGGGCATCGGTCGAGATCCCGGTGCCTAATGAGTGAGCTAACTTACATTAATTGCGTTGCGCTCACTGCCCGCTTTCCAGT
CGGGAAACCTGTCGTGCCAGCTGCATTAATGAATCGGCCAACGCGCGGGGAGAGGCGGTTTGCGTATTGGGCGCCAGGGTGGTTTTTCTTTTCA
CCAGTGAGACGGGCAACAGCTGATTGCCCTTCACCGCCTGGCCCTGAGAGAGTTGCAGCAAGCGGTCCACGCTGGTTTGCCCCAGCAGGCGAAA
ATCCTGTTTGATGGTGGTTAACGGCGGGATATAACATGAGCTGTCTTCGGTATCGTCGTATCCCACTACCGAGATATCCGCACCAACGCGCAGC
CCGGACTCGGTAATGGCGCGCATTGCGCCCAGCGCCATCTGATCGTTGGCAACCAGCATCGCAGTGGGAACGATGCCCTCATTCAGCATTTGCA
TGGTTTGTTGAAAACCGGACATGGCACTCCAGTCGCCTTCCCGTTCCGCTATCGGCTGAATTTGATTGCGAGTGAGATATTTATGCCAGCCAGC
CAGACGCAGACGCGCCGAGACAGAACTTAATGGGCCCGCTAACAGCGCGATTTGCTGGTGACCCAATGCGACCAGATGCTCCACGCCCAGTCGC
GTACCGTCTTCATGGGAGAAAATAATACTGTTGATGGGTGTCTGGTCAGAGACATCAAGAAATAACGCCGGAACATTAGTGCAGGCAGCTTCCA
CAGCAATGGCATCCTGGTCATCCAGCGGATAGTTAATGATCAGCCCACTGACGCGTTGCGCGAGAAGATTGTGCACCGCCGCTTTACAGGCTTC
GACGCCGCTTCGTTCTACCATCGACACCACCACGCTGGCACCCAGTTGATCGGCGCGAGATTTAATCGCCGCGACAATTTGCGACGGCGCGTGC
AGGGCCAGACTGGAGGTGGCAACGCCAATCAGCAACGACTGTTTGCCCGCCAGTTGTTGTGCCACGCGGTTGGGAATGTAATTCAGCTCCGCCA
TCGCCGCTTCCACTTTTTCCCGCGTTTTCGCAGAAACGTGGCTGGCCTGGTTCACCACGCGGGAAACGGTCTGATAAGAGACACCGGCATACTC
TGCGACATCGTATAACGTTACTGGTTTCACATTCACCACCCTGAATTGACTCTCTTCCGGGCGCTATCATGCCATACCGCGAAAGGTTTTGCGC
CATTCGATGGTGTCCGGGATCTCGACGCTCTCCCTTATGCGACTCCTGCATTAGGAAGCAGCCCAGTAGTAGGTTGAGGCCGTTGAGCACCGCC
GCCGCAAGGAATGGTGCATGCAAGGAGATGGCGCCCAACAGTCCCCCGGCCACGGGGCCTGCCACCATACCCACGCCGAAACAAGCGCTCATGA
GCCCGAAGTGGCGAGCCCGATCTTCCCCATCGGTGATGTCGGCGATATAGGCGCCAGCAACCGCACCTGTGGCGCCGGTGATGCCGGCCACGAT
GCGTCCGGCGTAGAGGATCGAGATCTCGATCCCGCGAAATTAATACGACTCACTATAGGGGAATTGTGAGCGGATAACAATTCCCCTCTAGAAA
TAATTTTGTTTAACTTTAAGAAGGAGATATACATATGAGCAAGCGTAAACTGCTGAAGAAAATCGAGAAGAAAGACACCAGCAGCGTGCTGACC
CAGAAGAAACAAACCAAGACCCTGTGGGCGGATGGCGTGCAGGTTGACGATAAGGACTTCAAACCGAGCACCGAAAACTTTGGTACCAACTACC
TGGCGGCGGAGTATGGTATCGGCAAGGGTTACTACGATATCAACAAGAAGTTCGACGGCACCGACGATGACCTGTGCAGCGGTGTGGTTGCGGC
GAACCAGCTGCACTGGTGGCTGGATCGTAACAAGGACTACATTGAAAAATATCGTCAGCAAAGCAAGGATAACGGCGTGACCATCGGTAACACC
GACATTTTCGAACTGAACAAACTGCACGATGAGGACCAGAGCAACTTCTTTGATTTCATCAAGAAAAGCTTCGGCAACAAGTTTCTGCAACCGG
AGCGTCTGCTGAACATGTACATTAACGGCTACGGTTATCTGACCAGCCAGGATAAGGCGAAAACCAGCCAACCGAGCCCGAGCAAGCTGAACTT
CTTTCAGAAGGTTTTCAAAAACAACCTGCTGACCGACAAAACCCCGATCAACGATATTGACGAATTTAGCAGCCAGACCAAGAACGCGCTGCAA
AACCACAAAGTGCTGGCGGTTAGCTTCGCGAGCATCAAGAACCGTGGCCTGGGTCACGTGGTTACCGTGTGGGGCGCGGATTTTGACGAGAACG
GTAAAGTGGTTGCGCTGTATGTTACCGACAGCGATGACCGTAGCAAGAACATTGGCAACGCGAAACTGGGTATGAAGAAACTGCGTATCGAAGT
GAGCGCGCAGGATAGCAGCACCATTAAGCTGACCGGCTTCGAGGACAAAAACAGCGGTGGCAGCCTGCGTCACCTGTACAGCCTGAGCACCGGT
GAACAAATCTGGAAGAAGTACTTCGAGGAAACCGAAAAGGAGCGTATTCGTCTGGAGGAAGAGGCGGATAAGGCGAAACTGGAACAGGACCGTA
TCCAAAAAGAGGCGGAAGAGAAACTGGCGCTGGAAAAGGCGGAAAAAGAGCGTATCCGTCTGGAAGAGGAAGCGGACAAAGCGAAGCTGGAACA
AGACCGTATTCAAAAAGAGGCGGAGGAAAAACTGGCGCTGGAGAAAGCGGAGAAGGAACGTATCCGCCTGGAGGAAGAGGCGGACAAAGCGAAA
CTGGAACAAGATCGCATTCAGAAAGAGGCGGAGGAGAAGCTGGCGCTGGAAAAAGCGGAGAAGGAGCGCATTCGCCTGGAAGAGGAAGCGGATA

AAGCGAAGCTGGAACAGGATCGCATCCAGAAAGAGGCGGAAGAGAAGCTGGCGCTGGAGAAGGCGGAGAAGGAGCGTATCCGACTGGAGGAAGA
GGCGGCGAAAGCTAAACTGGAACAAGAAAAACAAATTGCGACCGCGCCGCAGCCGGACAAGAAACAAGAAAACACCACCAGCGAACAGGAGAAA
CCGGCGCCGACCGAGCTGCCGCCGCTGGTTAACAAAGCGGATGAAACCGAGACCCCGCGTGAGACCGCGCCGGACCAAACCCCGAGCGCGACCA
ACACCTTCCGTAAGATCCTGCCGAAAATGAACGCTGTGAGCCAGTTCTTTAGCCAACTGATGGGTACCATCCAGATTGTTTTCGCGTTTATCCT
GAAGATTTTTAAAGGCAGCGGTCATCACCACCACCACCACTAACTCGAGCACCACCACCACCACCACTGAGATCCGGCTGCTAACAAAGCCCGA
AAGGAAGCTGAGTTGGCTGCTGCCACCGCTGAGCAATAACTAGCATAACCCCTTGGGGCCTCTAAACGGGTCTTGAGGGGTTTTTTGCTGAAAG
GAGGAACTATATCCGGAT

SEQ ID NO: 19 is an exemplary expression vector sequence comprising the
sequence of SEQ ID NO : 17. The vector is pET21a+. Gene is shaded grey.
TGGCGAATGGGACGCGCCCTGTAGCGGCGCATTAAGCGCGGCGGGTGTGGTGGTTACGCGCAGCGTGACCGCTACACTTGCCAGCGCCCTAGCG
CCCGCTCCTTTCGCTTTCTTCCCTTCCTTTCTCGCCACGTTCGCCGGCTTTCCCCGTCAAGCTCTAAATCGGGGGCTCCCTTTAGGGTTCCGAT
TTAGTGCTTTACGGCACCTCGACCCCAAAAAACTTGATTAGGGTGATGGTTCACGTAGTGGGCCATCGCCCTGATAGACGGTTTTTCGCCCTTT
GACGTTGGAGTCCACGTTCTTTAATAGTGGACTCTTGTTCCAAACTGGAACAACACTCAACCCTATCTCGGTCTATTCTTTTGATTTATAAGGG
ATTTTGCCGATTTCGGCCTATTGGTTAAAAAATGAGCTGATTTAACAAAAATTTAACGCGAATTTTAACAAAATATTAACGTTTACAATTTCAG
GTGGCACTTTTCGGGGAAATGTGCGCGGAACCCCTATTTGTTTATTTTTCTAAATACATTCAAATATGTATCCGCTCATGAGACAATAACCCTG
ATAAATGCTTCAATAATATTGAAAAAGGAAGAGTATGAGTATTCAACATTTCCGTGTCGCCCTTATTCCCTTTTTTGCGGCATTTTGCCTTCCT
GTTTTTGCTCACCCAGAAACGCTGGTGAAAGTAAAAGATGCTGAAGATCAGTTGGGTGCACGAGTGGGTTACATCGAACTGGATCTCAACAGCG
GTAAGATCCTTGAGAGTTTTCGCCCCGAAGAACGTTTTCCAATGATGAGCACTTTTAAAGTTCTGCTATGTGGCGCGGTATTATCCCGTATTGA
CGCCGGGCAAGAGCAACTCGGTCGCCGCATACACTATTCTCAGAATGACTTGGTTGAGTACTCACCAGTCACAGAAAAGCATCTTACGGATGGC
ATGACAGTAAGAGAATTATGCAGTGCTGCCATAACCATGAGTGATAACACTGCGGCCAACTTACTTCTGACAACGATCGGAGGACCGAAGGAGC
TAACCGCTTTTTTGCACAACATGGGGGATCATGTAACTCGCCTTGATCGTTGGGAACCGGAGCTGAATGAAGCCATACCAAACGACGAGCGTGA
CACCACGATGCCTGCAGCAATGGCAACAACGTTGCGCAAACTATTAACTGGCGAACTACTTACTCTAGCTTCCCGGCAACAATTAATAGACTGG
ATGGAGGCGGATAAAGTTGCAGGACCACTTCTGCGCTCGGCCCTTCCGGCTGGCTGGTTTATTGCTGATAAATCTGGAGCCGGTGAGCGTGGGT
CTCGCGGTATCATTGCAGCACTGGGGCCAGATGGTAAGCCCTCCCGTATCGTAGTTATCTACACGACGGGGAGTCAGGCAACTATGGATGAACG
AAATAGACAGATCGCTGAGATAGGTGCCTCACTGATTAAGCATTGGTAACTGTCAGACCAAGTTTACTCATATATACTTTAGATTGATTTAAAA
CTTCATTTTTAATTTAAAAGGATCTAGGTGAAGATCCTTTTTGATAATCTCATGACCAAAATCCCTTAACGTGAGTTTTCGTTCCACTGAGCGT
CAGACCCCGTAGAAAAGATCAAAGGATCTTCTTGAGATCCTTTTTTTCTGCGCGTAATCTGCTGCTTGCAAACAAAAAAACCACCGCTACCAGC
GGTGGTTTGTTTGCCGGATCAAGAGCTACCAACTCTTTTTCCGAAGGTAACTGGCTTCAGCAGAGCGCAGATACCAAATACTGTCCTTCTAGTG
TAGCCGTAGTTAGGCCACCACTTCAAGAACTCTGTAGCACCGCCTACATACCTCGCTCTGCTAATCCTGTTACCAGTGGCTGCTGCCAGTGGCG
ATAAGTCGTGTCTTACCGGGTTGGACTCAAGACGATAGTTACCGGATAAGGCGCAGCGGTCGGGCTGAACGGGGGGTTCGTGCACACAGCCCAG
CTTGGAGCGAACGACCTACACCGAACTGAGATACCTACAGCGTGAGCTATGAGAAAGCGCCACGCTTCCCGAAGGGAGAAAGGCGGACAGGTAT
CCGGTAAGCGGCAGGGTCGGAACAGGAGAGCGCACGAGGGAGCTTCCAGGGGGAAACGCCTGGTATCTTTATAGTCCTGTCGGGTTTCGCCACC
TCTGACTTGAGCGTCGATTTTTGTGATGCTCGTCAGGGGGGCGGAGCCTATGGAAAAACGCCAGCAACGCGGCCTTTTTACGGTTCCTGGCCTT
TTGCTGGCCTTTTGCTCACATGTTCTTTCCTGCGTTATCCCCTGATTCTGTGGATAACCGTATTACCGCCTTTGAGTGAGCTGATACCGCTCGC
CGCAGCCGAACGACCGAGCGCAGCGAGTCAGTGAGCGAGGAAGCGGAAGAGCGCCTGATGCGGTATTTTCTCCTTACGCATCTGTGCGGTATTT
CACACCGCATATATGGTGCACTCTCAGTACAATCTGCTCTGATGCCGCATAGTTAAGCCAGTATACACTCCGCTATCGCTACGTGACTGGGTCA
TGGCTGCGCCCCGACACCCGCCAACACCCGCTGACGCGCCCTGACGGGCTTGTCTGCTCCCGGCATCCGCTTACAGACAAGCTGTGACCGTCTC
CGGGAGCTGCATGTGTCAGAGGTTTTCACCGTCATCACCGAAACGCGCGAGGCAGCTGCGGTAAAGCTCATCAGCGTGGTCGTGAAGCGATTCA
CAGATGTCTGCCTGTTCATCCGCGTCCAGCTCGTTGAGTTTCTCCAGAAGCGTTAATGTCTGGCTTCTGATAAAGCGGGCCATGTTAAGGGCGG
TTTTTTCCTGTTTGGTCACTGATGCCTCCGTGTAAGGGGGATTTCTGTTCATGGGGGTAATGATACCGATGAAACGAGAGAGGATGCTCACGAT
ACGGGTTACTGATGATGAACATGCCCGGTTACTGGAACGTTGTGAGGGTAAACAACTGGCGGTATGGATGCGGCGGGACCAGAGAAAAATCACT
CAGGGTCAATGCCAGCGCTTCGTTAATACAGATGTAGGTGTTCCACAGGGTAGCCAGCAGCATCCTGCGATGCAGATCCGGAACATAATGGTGC
AGGGCGCTGACTTCCGCGTTTCCAGACTTTACGAAACACGGAAACCGAAGACCATTCATGTTGTTGCTCAGGTCGCAGACGTTTTGCAGCAGCA
GTCGCTTCACGTTCGCTCGCGTATCGGTGATTCATTCTGCTAACCAGTAAGGCAACCCCGCCAGCCTAGCCGGGTCCTCAACGACAGGAGCACG
ATCATGCGCACCCGTGGGGCCGCCATGCCGGCGATAATGGCCTGCTTCTCGCCGAAACGTTTGGTGGCGGGACCAGTGACGAAGGCTTGAGCGA
GGGCGTGCAAGATTCCGAATACCGCAAGCGACAGGCCGATCATCGTCGCGCTCCAGCGAAAGCGGTCCTCGCCGAAAATGACCCAGAGCGCTGC
CGGCACCTGTCCTACGAGTTGCATGATAAAGAAGACAGTCATAAGTGCGGCGACGATAGTCATGCCCCGCGCCCACCGGAAGGAGCTGACTGGG
TTGAAGGCTCTCAAGGGCATCGGTCGAGATCCCGGTGCCTAATGAGTGAGCTAACTTACATTAATTGCGTTGCGCTCACTGCCCGCTTTCCAGT
CGGGAAACCTGTCGTGCCAGCTGCATTAATGAATCGGCCAACGCGCGGGGAGAGGCGGTTTGCGTATTGGGCGCCAGGGTGGTTTTTCTTTTCA
CCAGTGAGACGGGCAACAGCTGATTGCCCTTCACCGCCTGGCCCTGAGAGAGTTGCAGCAAGCGGTCCACGCTGGTTTGCCCCAGCAGGCGAAA
ATCCTGTTTGATGGTGGTTAACGGCGGGATATAACATGAGCTGTCTTCGGTATCGTCGTATCCCACTACCGAGATATCCGCACCAACGCGCAGC
CCGGACTCGGTAATGGCGCGCATTGCGCCCAGCGCCATCTGATCGTTGGCAACCAGCATCGCAGTGGGAACGATGCCCTCATTCAGCATTTGCA
TGGTTTGTTGAAAACCGGACATGGCACTCCAGTCGCCTTCCCGTTCCGCTATCGGCTGAATTTGATTGCGAGTGAGATATTTATGCCAGCCAGC
CAGACGCAGACGCGCCGAGACAGAACTTAATGGGCCCGCTAACAGCGCGATTTGCTGGTGACCCAATGCGACCAGATGCTCCACGCCCAGTCGC
GTACCGTCTTCATGGGAGAAAATAATACTGTTGATGGGTGTCTGGTCAGAGACATCAAGAAATAACGCCGGAACATTAGTGCAGGCAGCTTCCA
CAGCAATGGCATCCTGGTCATCCAGCGGATAGTTAATGATCAGCCCACTGACGCGTTGCGCGAGAAGATTGTGCACCGCCGCTTTACAGGCTTC
GACGCCGCTTCGTTCTACCATCGACACCACCACGCTGGCACCCAGTTGATCGGCGCGAGATTTAATCGCCGCGACAATTTGCGACGGCGCGTGC
AGGGCCAGACTGGAGGTGGCAACGCCAATCAGCAACGACTGTTTGCCCGCCAGTTGTTGTGCCACGCGGTTGGGAATGTAATTCAGCTCCGCCA
TCGCCGCTTCCACTTTTTCCCGCGTTTTCGCAGAAACGTGGCTGGCCTGGTTCACCACGCGGGAAACGGTCTGATAAGAGACACCGGCATACTC
TGCGACATCGTATAACGTTACTGGTTTCACATTCACCACCCTGAATTGACTCTCTTCCGGGCGCTATCATGCCATACCGCGAAAGGTTTTGCGC
CATTCGATGGTGTCCGGGATCTCGACGCTCTCCCTTATGCGACTCCTGCATTAGGAAGCAGCCCAGTAGTAGGTTGAGGCCGTTGAGCACCGCC
GCCGCAAGGAATGGTGCATGCAAGGAGATGGCGCCCAACAGTCCCCCGGCCACGGGGCCTGCCACCATACCCACGCCGAAACAAGCGCTCATGA
GCCCGAAGTGGCGAGCCCGATCTTCCCCATCGGTGATGTCGGCGATATAGGCGCCAGCAACCGCACCTGTGGCGCCGGTGATGCCGGCCACGAT
GCGTCCGGCGTAGAGGATCGAGATCTCGATCCCGCGAAATTAATACGACTCACTATAGGGGAATTGTGAGCGGATAACAATTCCCCTCTAGAAA
TAATTTTGTTTAACTTTAAGAAGGAGATATACATATGAGCAAGCGTAAACTGCTGAAGAAAATCGAGAAGAAAGACACCAGCAGCGTGCTGACC
CAGAAGAAACAAACCAAGACCCTGTGGGCGGATGGCGTGCAGGTTGACGATAAGGACTTCAAACCGAGCACCGAAAACTTTGGTACCAACTACC
TGGCGGCGGAGTATGGTATCGGCAAGGGTTACTACGATATCAACAAGAAGTTCGACGGCACCGACGATGACCTGTGCAGCGGTGTGGTTGCGGC
GAACCAGCTGCACTGGTGGCTGGATCGTAACAAGGACTACATTGAAAAATATCGTCAGCAAAGCAAGGATAACGGCGTGACCATCGGTAACACC
GACATTTTCGAACTGAACAAACTGCACGATGAGGACCAGAGCAACTTCTTTGATTTCATCAAGAAAAGCTTCGGCAACAAGTTTCTGCAACCGG
AGCGTCTGCTGAACATGTACATTAACGGCTACGGTTATCTGACCAGCCAGGATAAGGCGAAAACCAGCCAACCGAGCCCGAGCAAGCTGAACTT
CTTTCAGAAGGTTTTCAAAAACAACCTGCTGACCGACAAAACCCCGATCAACGATATTGACGAATTTAGCAGCCAGACCAAGAACGCGCTGCAA
AACCACAAAGTGCTGGCGGTTAGCTTCGCGAGCATCAAGAACCGTGGCCTGGGTCACGTGGTTACCGTGTGGGGCGCGGATTTTGACGAGAACG
GTAAAGTGGTTGCGCTGTATGTTACCGACAGCGATGACCGTAGCAAGAACATTGGCAACGCGAAACTGGGTATGAAGAAACTGCGTATCGAAGT
GAGCGCGCAGGATAGCAGCACCATTAAGCTGACCGGCTTCGAGGACAAAAACAGCGGTGGCAGCCTGCGTCACCTGTACAGCCTGAGCACCGGT

GAACAAATCTGGAAGAAGTACTTCGAGGAAACCCATCACCACCACCACCACTAACTCGAGCACCACCACCACCACCACTGAGATCCGGCTGCTA
ACAAAGCCCGAAAGGAAGCTGAGTTGGCTGCTGCCACCGCTGAGCAATAACTAGCATAACCCCTTGGGGCCTCTAAACGGGTCTTGAGGGGTTT
TTTGCTGAAAGGAGGAACTATATCCGGAT

## Claims

1. A polypeptide, optionally an engineered polypeptide, having immunoglobulin protease activity, wherein said polypeptide comprises, consists essentially of, or consists of:
(a) the amino acid sequence of SEQ ID NO: 1;
(b) the amino acid sequence of SEQ ID NO: 2;
(c) an amino acid sequence which is an N terminal fragment of the sequence of SEQ ID NO: 1;
(d) an amino acid sequence which is at least 50% identical to the amino acid sequence of any one of (a), (b) or (c).

2. The polypeptide according to claim 1, is engineered to include an additional methionine at the N terminus and/or a protein purification or other tag at the C terminus, which tag may be joined to the C terminus by a linker.

3. The polypeptide according to claim 1 or 2 which comprises or consists of the amino acid sequence of SEQ ID NO: 3.

4. The polypeptide according to any one of the preceding claims which has protease activity against any immunoglobulin molecule comprising a CH2/hinge sequence as shown in any one of SEQ ID NOs: 4 to 8, wherein the polypeptide cleaves the said CH2/hinge sequence between the positions corresponding to positions 249 and 250 of human IgG according to the Kabat numbering system (positions 236 and 237 according to EU numbering system).

5. The polypeptide according to any one of the preceding claims which has protease activity against IgG, which may be human IgG1, IgG2, IgG3 or IgG4, mouse IgG2a or IgG3, or IgG from guinea pig or horse.

6. A polynucleotide or expression vector which comprises a nucleic acid sequence encoding a polypeptide of any one of the preceding claims.

7. A host cell comprising the polynucleotide or expression vector of claim 6, which is preferably a bacterial cell, preferably not a cell of a streptococcal species, and most preferably a cell of *E. coli.*

8. The polypeptide according to any one of claims 1 to 5, wherein the polypeptide is provided in solution, lyophilised, or immobilised, optionally together with an effective amount of at least one excipient, which is preferably a preservative.

9. A composition comprising a polypeptide according to any one of claims 1 to 5 together with at least one excipient, which is preferably a preservative; optionally wherein the composition is pharmaceutically acceptable.

10. A method comprising administering a polypeptide according to any one of claims 1 to 5 to a sample or a subject in which IgG is present.

11. The method of claim 10, which is for the ex vivo cleavage of IgG in a sample, and which comprises administering said polypeptide to the sample and incubating under conditions suitable for IgG protease activity.

12. The method of claim 11 which additionally comprises the separation, detection or analysis of the resulting cleavage products, and/or wherein the method generates Fc and Fab fragments.

13. The method of claim 10, which is for the prevention or treatment of a disease or condition in a subject, and which method comprises administering said polypeptide to the subject in a prophylactically or therapeutically effective amount.

14. The method of claim 13, wherein said disease or condition is a disease or condition mediated in whole or in part by pathogenic IgG antibodies.

15. The method of claim 13 or 14, wherein the disease or condition is (i) an autoimmune disease mediated in whole or in part by IgG antibodies; (ii) IgG mediated rejection of an organ or tissue transplant received by the subject; or (iii) IgG mediated anti-drug responses to a therapeutic agent administered to the subject.


SEQUENCE LISTING

<110>  GENOVIS AB

<120>  IMMUNOGLOBULIN CLEAVING ENZYME

<130>  N421594EP

<160>  19

<170>  PatentIn version 3.5

<210>  1
<211>  557
<212>  PRT
<213>  Streptococcus spp

<400>  1

Leu Ser Lys Arg Lys Leu Leu Lys Lys Ile Glu Lys Lys Asp Thr Ser
1               5                   10                  15

Ser Val Leu Thr Gln Lys Lys Gln Thr Lys Thr Leu Trp Ala Asp Gly
            20                  25                  30

Val Gln Val Asp Asp Lys Asp Phe Lys Pro Ser Thr Glu Asn Phe Gly
        35                  40                  45

Thr Asn Tyr Leu Ala Ala Glu Tyr Gly Ile Gly Lys Gly Tyr Tyr Asp
    50                  55                  60

Ile Asn Lys Lys Phe Asp Gly Thr Asp Asp Asp Leu Cys Ser Gly Val
65                  70                  75                  80

Val Ala Ala Asn Gln Leu His Trp Trp Leu Asp Arg Asn Lys Asp Tyr
                85                  90                  95

Ile Glu Lys Tyr Arg Gln Gln Ser Lys Asp Asn Gly Val Thr Ile Gly
            100                 105                 110

Asn Thr Asp Ile Phe Glu Leu Asn Lys Leu His Asp Glu Asp Gln Ser
        115                 120                 125

Asn Phe Phe Asp Phe Ile Lys Lys Ser Phe Gly Asn Lys Phe Leu Gln
    130                 135                 140

Pro Glu Arg Leu Leu Asn Met Tyr Ile Asn Gly Tyr Gly Tyr Leu Thr
145                 150                 155                 160

Ser Gln Asp Lys Ala Lys Thr Ser Gln Pro Ser Pro Ser Lys Leu Asn
                165                 170                 175

Phe Phe Gln Lys Val Phe Lys Asn Asn Leu Leu Thr Asp Lys Thr Pro
        180                 185                 190

Ile Asn Asp Ile Asp Glu Phe Ser Ser Gln Thr Lys Asn Ala Leu Gln
    195                 200                 205

Asn His Lys Val Leu Ala Val Ser Phe Ala Ser Ile Lys Asn Arg Gly
210                 215                 220

Leu Gly His Val Val Thr Val Trp Gly Ala Asp Phe Asp Glu Asn Gly
225                 230                 235                 240

Lys Val Val Ala Leu Tyr Val Thr Asp Ser Asp Asp Arg Ser Lys Asn
                245                 250                 255

Ile Gly Asn Ala Lys Leu Gly Met Lys Lys Leu Arg Ile Glu Val Ser
            260                 265                 270

Ala Gln Asp Ser Ser Thr Ile Lys Leu Thr Gly Phe Glu Asp Lys Asn
        275                 280                 285

Ser Gly Gly Ser Leu Arg His Leu Tyr Ser Leu Ser Thr Gly Glu Gln
    290                 295                 300

Ile Trp Lys Lys Tyr Phe Glu Glu Thr Glu Lys Glu Arg Ile Arg Leu
305                 310                 315                 320

Glu Glu Glu Ala Asp Lys Ala Lys Leu Glu Gln Asp Arg Ile Gln Lys
                325                 330                 335

Glu Ala Glu Glu Lys Leu Ala Leu Glu Lys Ala Glu Lys Glu Arg Ile
            340                 345                 350

Arg Leu Glu Glu Glu Ala Asp Lys Ala Lys Leu Glu Gln Asp Arg Ile
        355                 360                 365

Gln Lys Glu Ala Glu Glu Lys Leu Ala Leu Glu Lys Ala Glu Lys Glu
    370                 375                 380

Arg Ile Arg Leu Glu Glu Glu Ala Asp Lys Ala Lys Leu Glu Gln Asp
385                 390                 395                 400

Arg Ile Gln Lys Glu Ala Glu Glu Lys Leu Ala Leu Glu Lys Ala Glu
                405                 410                 415

Lys Glu Arg Ile Arg Leu Glu Glu Glu Ala Asp Lys Ala Lys Leu Glu
            420                 425                 430

Gln Asp Arg Ile Gln Lys Glu Ala Glu Glu Lys Leu Ala Leu Glu Lys
        435                 440                 445

Ala Glu Lys Glu Arg Ile Arg Leu Glu Glu Glu Ala Ala Lys Ala Lys
    450                 455                 460

Leu Glu Gln Glu Lys Gln Ile Ala Thr Ala Pro Gln Pro Asp Lys Lys
465                 470                 475                 480

Gln Glu Asn Thr Thr Ser Glu Gln Glu Lys Pro Ala Pro Thr Glu Leu
                485                 490                 495

Pro Pro Leu Val Asn Lys Ala Asp Glu Thr Glu Thr Pro Arg Glu Thr
            500                 505                 510

Ala Pro Asp Gln Thr Pro Ser Ala Thr Asn Thr Phe Arg Lys Ile Leu
        515                 520                 525

Pro Lys Met Asn Ala Val Ser Gln Phe Phe Ser Gln Leu Met Gly Thr
    530                 535                 540

Ile Gln Ile Val Phe Ala Phe Ile Leu Lys Ile Phe Lys
545                 550                 555

<210>  2
<211>  312
<212>  PRT
<213>  Artificial Sequence

<220>
<223>  polypeptide

<400>  2

Ser Lys Arg Lys Leu Leu Lys Lys Ile Glu Lys Lys Asp Thr Ser Ser
1               5                   10                  15

Val Leu Thr Gln Lys Lys Gln Thr Lys Thr Leu Trp Ala Asp Gly Val
            20                  25                  30

Gln Val Asp Asp Lys Asp Phe Lys Pro Ser Thr Glu Asn Phe Gly Thr
        35                  40                  45

Asn Tyr Leu Ala Ala Glu Tyr Gly Ile Gly Lys Gly Tyr Tyr Asp Ile
    50                  55                  60

Asn Lys Lys Phe Asp Gly Thr Asp Asp Asp Leu Cys Ser Gly Val Val
65                  70                  75                  80

Ala Ala Asn Gln Leu His Trp Trp Leu Asp Arg Asn Lys Asp Tyr Ile
                85                  90                  95

Glu Lys Tyr Arg Gln Gln Ser Lys Asp Asn Gly Val Thr Ile Gly Asn
            100                 105                 110

Thr Asp Ile Phe Glu Leu Asn Lys Leu His Asp Glu Asp Gln Ser Asn
        115                 120                 125

Phe Phe Asp Phe Ile Lys Lys Ser Phe Gly Asn Lys Phe Leu Gln Pro
    130                 135                 140

Glu Arg Leu Leu Asn Met Tyr Ile Asn Gly Tyr Gly Tyr Leu Thr Ser
145                 150                 155                 160

Gln Asp Lys Ala Lys Thr Ser Gln Pro Ser Pro Ser Lys Leu Asn Phe
                165                 170                 175

Phe Gln Lys Val Phe Lys Asn Asn Leu Leu Thr Asp Lys Thr Pro Ile
            180                 185                 190

Asn Asp Ile Asp Glu Phe Ser Ser Gln Thr Lys Asn Ala Leu Gln Asn
        195                 200                 205

His Lys Val Leu Ala Val Ser Phe Ala Ser Ile Lys Asn Arg Gly Leu
    210                 215                 220

Gly His Val Val Thr Val Trp Gly Ala Asp Phe Asp Glu Asn Gly Lys
225                 230                 235                 240

Val Val Ala Leu Tyr Val Thr Asp Ser Asp Asp Arg Ser Lys Asn Ile
                245                 250                 255

Gly Asn Ala Lys Leu Gly Met Lys Lys Leu Arg Ile Glu Val Ser Ala
            260                 265                 270

Gln Asp Ser Ser Thr Ile Lys Leu Thr Gly Phe Glu Asp Lys Asn Ser
        275                 280                 285

Gly Gly Ser Leu Arg His Leu Tyr Ser Leu Ser Thr Gly Glu Gln Ile
    290                 295                 300

Trp Lys Lys Tyr Phe Glu Glu Thr
305                 310

<210>  3
<211>  319
<212>  PRT

<213>  Artificial Sequence

<220>
<223>  polypeptide

<400>  3

Met Ser Lys Arg Lys Leu Leu Lys Lys Ile Glu Lys Lys Asp Thr Ser
1               5                   10                  15

Ser Val Leu Thr Gln Lys Lys Gln Thr Lys Thr Leu Trp Ala Asp Gly
            20                  25                  30

Val Gln Val Asp Asp Lys Asp Phe Lys Pro Ser Thr Glu Asn Phe Gly
        35                  40                  45

Thr Asn Tyr Leu Ala Ala Glu Tyr Gly Ile Gly Lys Gly Tyr Tyr Asp
    50                  55                  60

Ile Asn Lys Lys Phe Asp Gly Thr Asp Asp Asp Leu Cys Ser Gly Val
65                  70                  75                  80

Val Ala Ala Asn Gln Leu His Trp Trp Leu Asp Arg Asn Lys Asp Tyr
                85                  90                  95

Ile Glu Lys Tyr Arg Gln Gln Ser Lys Asp Asn Gly Val Thr Ile Gly
            100                 105                 110

Asn Thr Asp Ile Phe Glu Leu Asn Lys Leu His Asp Glu Asp Gln Ser
        115                 120                 125

Asn Phe Phe Asp Phe Ile Lys Lys Ser Phe Gly Asn Lys Phe Leu Gln
    130                 135                 140

Pro Glu Arg Leu Leu Asn Met Tyr Ile Asn Gly Tyr Gly Tyr Leu Thr
145                 150                 155                 160

Ser Gln Asp Lys Ala Lys Thr Ser Gln Pro Ser Pro Ser Lys Leu Asn
                165                 170                 175

Phe Phe Gln Lys Val Phe Lys Asn Asn Leu Leu Thr Asp Lys Thr Pro
            180                 185                 190

Ile Asn Asp Ile Asp Glu Phe Ser Ser Gln Thr Lys Asn Ala Leu Gln
        195                 200                 205

Asn His Lys Val Leu Ala Val Ser Phe Ala Ser Ile Lys Asn Arg Gly
    210                 215                 220

Leu Gly His Val Val Thr Val Trp Gly Ala Asp Phe Asp Glu Asn Gly
225                 230                 235                 240

Lys Val Val Ala Leu Tyr Val Thr Asp Ser Asp Asp Arg Ser Lys Asn
                245                 250                 255

Ile Gly Asn Ala Lys Leu Gly Met Lys Lys Leu Arg Ile Glu Val Ser
            260                 265                 270

Ala Gln Asp Ser Ser Thr Ile Lys Leu Thr Gly Phe Glu Asp Lys Asn
        275                 280                 285

Ser Gly Gly Ser Leu Arg His Leu Tyr Ser Leu Ser Thr Gly Glu Gln
    290                 295                 300

Ile Trp Lys Lys Tyr Phe Glu Glu Thr His His His His His His
305                 310                 315

<210>  4
<211>  16
<212>  PRT
<213>  Artificial Sequence

<220>
<223>  cleavage site

<400>  4

Cys Pro Pro Cys Pro Ala Pro Glu Leu Leu Gly Gly Pro Ser Val Phe
1               5                   10                  15

<210>  5
<211>  15
<212>  PRT
<213>  Artificial Sequence

<220>
<223>  cleavage site

<400>  5

Cys Pro Pro Cys Pro Ala Pro Pro Val Ala Gly Pro Ser Val Phe
1               5                   10                  15

<210>  6
<211>  16
<212>  PRT
<213>  Artificial Sequence

<220>
<223>  cleavage site

<400>  6

Cys Pro Arg Cys Pro Ala Pro Glu Leu Leu Gly Gly Pro Ser Val Phe

1               5                   10                  15


<210>  7
<211>  16
<212>  PRT
<213>  Artificial Sequence

<220>
<223>  cleaveage site

<400>  7

Ala His His Ala Gln Ala Pro Glu Phe Leu Gly Gly Pro Ser Val Phe
1               5                   10                  15


<210>  8
<211>  16
<212>  PRT
<213>  Artificial Sequence

<220>
<223>  cleavage site

<400>  8

Cys Pro Pro Cys Ala Ala Pro Asn Leu Leu Gly Gly Pro Ser Val Phe
1               5                   10                  15


<210>  9
<211>  16
<212>  PRT
<213>  Artificial Sequence

<220>
<223>  cleavage site

<400>  9

Gly Ser Ser Cys Pro Ala Gly Asn Ile Leu Gly Gly Pro Ser Val Phe
1               5                   10                  15


<210>  10
<211>  13
<212>  PRT
<213>  Artificial Sequence

<220>
<223>  cleavage site

<400>  10

Pro Cys Ile Cys Thr Val Pro Glu Val Ser Ser Val Phe
1               5                   10


<210>  11
<211>  16
<212>  PRT
<213>  Artificial Sequence

<220>
<223>  cleavage site

<400>  11

Cys His Lys Cys Pro Ala Pro Asn Leu Glu Gly Gly Pro Ser Val Phe
1               5                   10                  15


<210>  12
<211>  339
<212>  PRT
<213>  Streptococcus pyogenes

<400>  12

Met Arg Lys Arg Cys Tyr Ser Thr Ser Ala Ala Val Leu Ala Ala Val
1               5                   10                  15

Thr Leu Phe Val Leu Ser Val Asp Arg Gly Val Ile Ala Asp Ser Phe
            20                  25                  30

Ser Ala Asn Gln Glu Ile Arg Tyr Ser Glu Val Thr Pro Tyr His Val
        35                  40                  45

Thr Ser Val Trp Thr Lys Gly Val Thr Pro Pro Ala Asn Phe Thr Gln
    50                  55                  60

Gly Glu Asp Val Phe His Ala Pro Tyr Val Ala Asn Gln Gly Trp Tyr
65                  70                  75                  80

Asp Ile Thr Lys Thr Phe Asn Gly Lys Asp Asp Leu Leu Cys Gly Ala
                85                  90                  95

Ala Thr Ala Gly Asn Met Leu His Trp Trp Phe Asp Gln Asn Lys Asp
            100                 105                 110

Gln Ile Lys Arg Tyr Leu Glu Glu His Pro Glu Lys Gln Lys Ile Asn
        115                 120                 125

Phe Asn Gly Glu Gln Met Phe Asp Val Lys Glu Ala Ile Asp Thr Lys
    130                 135                 140

Asn His Gln Leu Asp Ser Lys Leu Phe Glu Tyr Phe Lys Glu Lys Ala
145                 150                 155                 160

Phe Pro Tyr Leu Ser Thr Lys His Leu Gly Val Phe Pro Asp His Val
                165                 170                 175

Ile Asp Met Phe Ile Asn Gly Tyr Arg Leu Ser Leu Thr Asn His Gly
            180                 185                 190

Pro Thr Pro Val Lys Glu Gly Ser Lys Asp Pro Arg Gly Gly Ile Phe
        195                 200                 205

Asp Ala Val Phe Thr Arg Gly Asp Gln Ser Lys Leu Leu Thr Ser Arg
    210                 215                 220

His Asp Phe Lys Glu Lys Asn Leu Lys Glu Ile Ser Asp Leu Ile Lys
225                 230                 235                 240

Lys Glu Leu Thr Glu Gly Lys Ala Leu Gly Leu Ser His Thr Tyr Ala
                245                 250                 255

Asn Val Arg Ile Asn His Val Ile Asn Leu Trp Gly Ala Asp Phe Asp
            260                 265                 270

Ser Asn Gly Asn Leu Lys Ala Ile Tyr Val Thr Asp Ser Asp Ser Asn
        275                 280                 285

Ala Ser Ile Gly Met Lys Lys Tyr Phe Val Gly Val Asn Ser Ala Gly
    290                 295                 300

Lys Val Ala Ile Ser Ala Lys Glu Ile Lys Glu Asp Asn Ile Gly Ala
305                 310                 315                 320

Gln Val Leu Gly Leu Phe Thr Leu Ser Thr Gly Gln Asp Ser Trp Asn
                325                 330                 335

Gln Thr Asn

<210>  13
<211>  310
<212>  PRT
<213>  Streptococcus pyogenes

<400>  13

Asp Ser Phe Ser Ala Asn Gln Glu Ile Arg Tyr Ser Glu Val Thr Pro
1               5                   10                  15

Tyr His Val Thr Ser Val Trp Thr Lys Gly Val Thr Pro Pro Ala Asn
            20                  25                  30

Phe Thr Gln Gly Glu Asp Val Phe His Ala Pro Tyr Val Ala Asn Gln
        35                  40                  45

Gly Trp Tyr Asp Ile Thr Lys Thr Phe Asn Gly Lys Asp Asp Leu Leu
    50                  55                  60

Cys Gly Ala Ala Thr Ala Gly Asn Met Leu His Trp Trp Phe Asp Gln
65                  70                  75                  80

Asn Lys Asp Gln Ile Lys Arg Tyr Leu Glu Glu His Pro Glu Lys Gln
                85                  90                  95

Lys Ile Asn Phe Asn Gly Glu Gln Met Phe Asp Val Lys Glu Ala Ile
            100                 105                 110

Asp Thr Lys Asn His Gln Leu Asp Ser Lys Leu Phe Glu Tyr Phe Lys
        115                 120                 125

Glu Lys Ala Phe Pro Tyr Leu Ser Thr Lys His Leu Gly Val Phe Pro
    130                 135                 140

Asp His Val Ile Asp Met Phe Ile Asn Gly Tyr Arg Leu Ser Leu Thr
145                 150                 155                 160

Asn His Gly Pro Thr Pro Val Lys Glu Gly Ser Lys Asp Pro Arg Gly
                165                 170                 175

Gly Ile Phe Asp Ala Val Phe Thr Arg Gly Asp Gln Ser Lys Leu Leu
            180                 185                 190

Thr Ser Arg His Asp Phe Lys Glu Lys Asn Leu Lys Glu Ile Ser Asp
        195                 200                 205

Leu Ile Lys Lys Glu Leu Thr Glu Gly Lys Ala Leu Gly Leu Ser His
    210                 215                 220

Thr Tyr Ala Asn Val Arg Ile Asn His Val Ile Asn Leu Trp Gly Ala
225                 230                 235                 240

Asp Phe Asp Ser Asn Gly Asn Leu Lys Ala Ile Tyr Val Thr Asp Ser
                245                 250                 255

Asp Ser Asn Ala Ser Ile Gly Met Lys Lys Tyr Phe Val Gly Val Asn
            260                 265                 270

Ser Ala Gly Lys Val Ala Ile Ser Ala Lys Glu Ile Lys Glu Asp Asn
        275                 280                 285

Ile Gly Ala Gln Val Leu Gly Leu Phe Thr Leu Ser Thr Gly Gln Asp
    290                 295                 300

Ser Trp Asn Gln Thr Asn

305                 310

<210>  14
<211>  349
<212>  PRT
<213>  Streptococcus equi

<400>  14

Met Lys Thr Ile Ala Tyr Pro Asn Lys Pro His Ser Leu Ser Ala Gly
1               5                   10                  15

Leu Leu Thr Ala Ile Ala Ile Phe Ser Leu Ala Ser Ser Asn Ile Thr
            20                  25                  30

Tyr Ala Asp Asp Tyr Gln Arg Asn Ala Thr Glu Ala Tyr Ala Lys Glu
        35                  40                  45

Val Pro His Gln Ile Thr Ser Val Trp Thr Lys Gly Val Thr Pro Leu
    50                  55                  60

Thr Pro Glu Gln Phe Arg Tyr Asn Asn Glu Asp Val Ile His Ala Pro
65                  70                  75                  80

Tyr Leu Ala His Gln Gly Trp Tyr Asp Ile Thr Lys Ala Phe Asp Gly
                85                  90                  95

Lys Asp Asn Leu Leu Cys Gly Ala Ala Thr Ala Gly Asn Met Leu His
            100                 105                 110

Trp Trp Phe Asp Gln Asn Lys Thr Glu Ile Glu Ala Tyr Leu Ser Lys
        115                 120                 125

His Pro Glu Lys Gln Lys Ile Ile Phe Asn Asn Gln Glu Leu Phe Asp
    130                 135                 140

Leu Lys Ala Ala Ile Asp Thr Lys Asp Ser Gln Thr Asn Ser Gln Leu
145                 150                 155                 160

Phe Asn Tyr Phe Arg Asp Lys Ala Phe Pro Asn Leu Ser Ala Arg Gln
                165                 170                 175

Leu Gly Val Met Pro Asp Leu Val Leu Asp Met Phe Ile Asn Gly Tyr
            180                 185                 190

Tyr Leu Asn Val Phe Lys Thr Gln Ser Thr Asp Val Asn Arg Pro Tyr
        195                 200                 205

Gln Asp Lys Asp Lys Arg Gly Gly Ile Phe Asp Ala Val Phe Thr Arg

210                 215                 220

Gly Asp Gln Thr Thr Leu Leu Thr Ala Arg His Asp Leu Lys Asn Lys
225                 230                 235                 240

Gly Leu Asn Asp Ile Ser Thr Ile Ile Lys Gln Glu Leu Thr Glu Gly
                245                 250                 255

Arg Ala Leu Ala Leu Ser His Thr Tyr Ala Asn Val Ser Ile Ser His
            260                 265                 270

Val Ile Asn Leu Trp Gly Ala Asp Phe Asn Ala Glu Gly Asn Leu Glu
        275                 280                 285

Ala Ile Tyr Val Thr Asp Ser Asp Ala Asn Ala Ser Ile Gly Met Lys
    290                 295                 300

Lys Tyr Phe Val Gly Ile Asn Ala His Gly His Val Ala Ile Ser Ala
305                 310                 315                 320

Lys Lys Ile Glu Gly Glu Asn Ile Gly Ala Gln Val Leu Gly Leu Phe
                325                 330                 335

Thr Leu Ser Ser Gly Lys Asp Ile Trp Gln Lys Leu Ser
            340                 345

<210>  15
<211>  315
<212>  PRT
<213>  Streptococcus equi

<400>  15

Asp Asp Tyr Gln Arg Asn Ala Thr Glu Ala Tyr Ala Lys Glu Val Pro
1               5                   10                  15

His Gln Ile Thr Ser Val Trp Thr Lys Gly Val Thr Pro Leu Thr Pro
            20                  25                  30

Glu Gln Phe Arg Tyr Asn Asn Glu Asp Val Ile His Ala Pro Tyr Leu
        35                  40                  45

Ala His Gln Gly Trp Tyr Asp Ile Thr Lys Ala Phe Asp Gly Lys Asp
    50                  55                  60

Asn Leu Leu Cys Gly Ala Ala Thr Ala Gly Asn Met Leu His Trp Trp
65                  70                  75                  80

Phe Asp Gln Asn Lys Thr Glu Ile Glu Ala Tyr Leu Ser Lys His Pro

85                  90                  95

Glu Lys Gln Lys Ile Ile Phe Asn Asn Gln Glu Leu Phe Asp Leu Lys
            100                 105                 110

Ala Ala Ile Asp Thr Lys Asp Ser Gln Thr Asn Ser Gln Leu Phe Asn
        115                 120                 125

Tyr Phe Arg Asp Lys Ala Phe Pro Asn Leu Ser Ala Arg Gln Leu Gly
    130                 135                 140

Val Met Pro Asp Leu Val Leu Asp Met Phe Ile Asn Gly Tyr Tyr Leu
145                 150                 155                 160

Asn Val Phe Lys Thr Gln Ser Thr Asp Val Asn Arg Pro Tyr Gln Asp
                165                 170                 175

Lys Asp Lys Arg Gly Gly Ile Phe Asp Ala Val Phe Thr Arg Gly Asp
            180                 185                 190

Gln Thr Thr Leu Leu Thr Ala Arg His Asp Leu Lys Asn Lys Gly Leu
        195                 200                 205

Asn Asp Ile Ser Thr Ile Ile Lys Gln Glu Leu Thr Glu Gly Arg Ala
    210                 215                 220

Leu Ala Leu Ser His Thr Tyr Ala Asn Val Ser Ile Ser His Val Ile
225                 230                 235                 240

Asn Leu Trp Gly Ala Asp Phe Asn Ala Glu Gly Asn Leu Glu Ala Ile
                245                 250                 255

Tyr Val Thr Asp Ser Asp Ala Asn Ala Ser Ile Gly Met Lys Lys Tyr
            260                 265                 270

Phe Val Gly Ile Asn Ala His Gly His Val Ala Ile Ser Ala Lys Lys
        275                 280                 285

Ile Glu Gly Glu Asn Ile Gly Ala Gln Val Leu Gly Leu Phe Thr Leu
    290                 295                 300

Ser Ser Gly Lys Asp Ile Trp Gln Lys Leu Ser
305                 310                 315

<210>  16
<211>  1701
<212>  DNA
<213>  Artificial Sequence

<220>
<223>  polynucleotide

<400>  16
atgagcaagc gtaaactgct gaagaaaatc gagaagaaag acaccagcag cgtgctgacc      60

cagaagaaac aaaccaagac cctgtgggcg gatggcgtgc aggttgacga taaggacttc     120

aaaccgagca ccgaaaactt tggtaccaac tacctggcgg cggagtatgg tatcggcaag     180

ggttactacg atatcaacaa gaagttcgac ggcaccgacg atgacctgtg cagcggtgtg     240

gttgcggcga accagctgca ctggtggctg gatcgtaaca aggactacat tgaaaaatat     300

cgtcagcaaa gcaaggataa cggcgtgacc atcggtaaca ccgacatttt cgaactgaac     360

aaactgcacg atgaggacca gagcaacttc tttgatttca tcaagaaaag cttcggcaac     420

aagtttctgc aaccggagcg tctgctgaac atgtacatta acggctacgg ttatctgacc     480

agccaggata aggcgaaaac cagccaaccg agcccgagca agctgaactt ctttcagaag     540

gttttcaaaa acaacctgct gaccgacaaa accccgatca acgatattga cgaatttagc     600

agccagacca agaacgcgct gcaaaaccac aaagtgctgg cggttagctt cgcgagcatc     660

aagaaccgtg gcctgggtca cgtggttacc gtgtggggcg cggattttga cgagaacggt     720

aaagtggttg cgctgtatgt taccgacagc gatgaccgta gcaagaacat tggcaacgcg     780

aaactgggta tgaagaaact gcgtatcgaa gtgagcgcgc aggatagcag caccattaag     840

ctgaccggct tcgaggacaa aaacagcggt ggcagcctgc gtcacctgta cagcctgagc     900

accggtgaac aaatctggaa gaagtacttc gaggaaaccg aaaaggagcg tattcgtctg     960

gaggaagagg cggataaggc gaaactggaa caggaccgta tccaaaaaga ggcggaagag    1020

aaactggcgc tggaaaaggc ggaaaaagag cgtatccgtc tggaagagga agcggacaaa    1080

gcgaagctgg aacaagaccg tattcaaaaa gaggcggagg aaaaactggc gctggagaaa    1140

gcggagaagg aacgtatccg cctggaggaa gaggcggaca aagcgaaact ggaacaagat    1200

cgcattcaga aagaggcgga ggagaagctg gcgctggaaa aagcggagaa ggagcgcatt    1260

cgcctggaag aggaagcgga taaagcgaag ctggaacagg atcgcatcca gaaagaggcg    1320

gaagagaagc tggcgctgga gaaggcggag aaggagcgta tccgactgga ggaagaggcg    1380

gcgaaagcta aactggaaca agaaaaacaa attgcgaccg cgccgcagcc ggacaagaaa    1440

caagaaaaca ccaccagcga acaggagaaa ccggcgccga ccgagctgcc gccgctggtt    1500

aacaaagcgg atgaaaccga gaccccgcgt gagaccgcgc cggaccaaac cccgagcgcg    1560

accaacacct tccgtaagat cctgccgaaa atgaacgctg tgagccagtt ctttagccaa    1620

ctgatgggta ccatccagat tgttttcgcg tttatcctga agatttttaa aggcagcggt    1680

catcaccacc accaccacta a                                              1701

<210>  17
<211>  918
<212>  DNA
<213>  Artifical Sequence

<220>
<223>  polynucleotide

<400>  17
atgagcaagc gtaaactgct gaagaaaatc gagaagaaag acaccagcag cgtgctgacc      60

cagaagaaac aaaccaagac cctgtgggcg gatggcgtgc aggttgacga taaggacttc     120

aaaccgagca ccgaaaactt tggtaccaac tacctggcgg cggagtatgg tatcggcaag     180

ggttactacg atatcaacaa gaagttcgac ggcaccgacg atgacctgtg cagcggtgtg     240

gttgcggcga accagctgca ctggtggctg gatcgtaaca aggactacat tgaaaaatat     300

cgtcagcaaa gcaaggataa cggcgtgacc atcggtaaca ccgacatttt cgaactgaac     360

aaactgcacg atgaggacca gagcaacttc tttgatttca tcaagaaaag cttcggcaac     420

aagtttctgc aaccggagcg tctgctgaac atgtacatta acggctacgg ttatctgacc     480

agccaggata aggcgaaaac cagccaaccg agcccgagca agctgaactt ctttcagaag     540

gttttcaaaa acaacctgct gaccgacaaa accccgatca acgatattga cgaatttagc     600

agccagacca agaacgcgct gcaaaaccac aaagtgctgg cggttagctt cgcgagcatc     660

aagaaccgtg gcctgggtca cgtggttacc gtgtggggcg cggattttga cgagaacggt     720

aaagtggttg cgctgtatgt taccgacagc gatgaccgta gcaagaacat tggcaacgcg     780

aaactgggta tgaagaaact gcgtatcgaa gtgagcgcgc aggatagcag caccattaag     840

ctgaccggct tcgaggacaa aaacagcggt ggcagcctgc gtcacctgta cagcctgcat     900

caccaccacc accactaa                                                   918


<210>  18
<211>  7068
<212>  DNA
<213>  Artificial Sequence

<220>
<223>  vector

<400>  18
tggcgaatgg gacgcgccct gtagcggcgc attaagcgcg gcgggtgtgg tggttacgcg      60

cagcgtgacc gctacacttg ccagcgccct agcgcccgct cctttcgctt tcttcccttc     120

ctttctcgcc acgttcgccg gctttccccg tcaagctcta aatcgggggc tccctttagg     180

gttccgattt agtgctttac ggcacctcga ccccaaaaaa cttgattagg gtgatggttc     240

acgtagtggg ccatcgccct gatagacggt ttttcgccct ttgacgttgg agtccacgtt     300

ctttaatagt ggactcttgt tccaaactgg aacaacactc aaccctatct cggtctattc     360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg agacaataac cctgataaat gcttcaataa tattgaaaaa ggaagagtat 600

gagtattcaa catttccgtg tcgcccttat tccctttttt gcggcatttt gccttcctgt 660

ttttgctcac ccagaaacgc tggtgaaagt aaaagatgct gaagatcagt tgggtgcacg 720

agtgggttac atcgaactgg atctcaacag cggtaagatc cttgagagtt ttcgccccga 780

agaacgtttt ccaatgatga gcacttttaa agttctgcta tgtggcgcgg tattatcccg 840

tattgacgcc gggcaagagc aactcggtcg ccgcatacac tattctcaga atgacttggt 900

tgagtactca ccagtcacag aaaagcatct tacggatggc atgacagtaa gagaattatg 960

cagtgctgcc ataaccatga gtgataacac tgcggccaac ttacttctga caacgatcgg 1020

aggaccgaag gagctaaccg cttttttgca caacatgggg gatcatgtaa ctcgccttga 1080

tcgttgggaa ccggagctga atgaagccat accaaacgac gagcgtgaca ccacgatgcc 1140

tgcagcaatg gcaacaacgt tgcgcaaact attaactggc gaactactta ctctagcttc 1200

ccggcaacaa ttaatagact ggatggaggc ggataaagtt gcaggaccac ttctgcgctc 1260

ggcccttccg gctggctggt ttattgctga taaatctgga gccggtgagc gtgggtctcg 1320

cggtatcatt gcagcactgg ggccagatgg taagccctcc cgtatcgtag ttatctacac 1380

gacggggagt caggcaacta tggatgaacg aaatagacag atcgctgaga taggtgcctc 1440

actgattaag cattggtaac tgtcagacca agtttactca tatatacttt agattgattt 1500

aaaacttcat ttttaattta aaaggatcta ggtgaagatc ctttttgata atctcatgac 1560

caaaatccct taacgtgagt tttcgttcca ctgagcgtca gaccccgtag aaaagatcaa 1620

aggatcttct tgagatcctt tttttctgcg cgtaatctgc tgcttgcaaa caaaaaaacc 1680

accgctacca gcggtggttt gtttgccgga tcaagagcta ccaactcttt ttccgaaggt 1740

aactggcttc agcagagcgc agataccaaa tactgtcctt ctagtgtagc cgtagttagg 1800

ccaccacttc aagaactctg tagcaccgcc tacatacctc gctctgctaa tcctgttacc 1860

agtggctgct gccagtggcg ataagtcgtg tcttaccggg ttggactcaa gacgatagtt 1920

accggataag gcgcagcggt cgggctgaac ggggggttcg tgcacacagc ccagcttgga 1980

gcgaacgacc tacaccgaac tgagatacct acagcgtgag ctatgagaaa gcgccacgct 2040

tcccgaaggg agaaaggcgg acaggtatcc ggtaagcggc agggtcggaa caggagagcg 2100

cacgagggag cttccagggg gaaacgcctg gtatctttat agtcctgtcg ggtttcgcca 2160

cctctgactt gagcgtcgat ttttgtgatg ctcgtcaggg gggcggagcc tatggaaaaa 2220

cgccagcaac gcggcctttt tacggttcct ggccttttgc tggccttttg ctcacatgtt 2280

ctttcctgcg ttatcccctg attctgtgga taaccgtatt accgcctttg agtgagctga 2340

taccgctcgc cgcagccgaa cgaccgagcg cagcgagtca gtgagcgagg aagcggaaga 2400

gcgcctgatg cggtattttc tccttacgca tctgtgcggt atttcacacc gcatatatgg 2460

tgcactctca gtacaatctg ctctgatgcc gcatagttaa gccagtatac actccgctat 2520

cgctacgtga ctgggtcatg gctgcgcccc gacacccgcc aacacccgct gacgcgccct 2580

gacgggcttg tctgctcccg gcatccgctt acagacaagc tgtgaccgtc tccgggagct 2640

gcatgtgtca gaggttttca ccgtcatcac cgaaacgcgc gaggcagctg cggtaaagct 2700

catcagcgtg gtcgtgaagc gattcacaga tgtctgcctg ttcatccgcg tccagctcgt 2760

tgagtttctc cagaagcgtt aatgtctggc ttctgataaa gcgggccatg ttaagggcgg 2820

ttttttcctg tttggtcact gatgcctccg tgtaaggggg atttctgttc atgggggtaa 2880

tgataccgat gaaacgagag aggatgctca cgatacgggt tactgatgat gaacatgccc 2940

ggttactgga acgttgtgag ggtaaacaac tggcggtatg gatgcggcgg gaccagagaa 3000

aaatcactca gggtcaatgc cagcgcttcg ttaatacaga tgtaggtgtt ccacagggta 3060

gccagcagca tcctgcgatg cagatccgga acataatggt gcagggcgct gacttccgcg 3120

tttccagact ttacgaaaca cggaaaccga agaccattca tgttgttgct caggtcgcag 3180

acgttttgca gcagcagtcg cttcacgttc gctcgcgtat cggtgattca ttctgctaac 3240

cagtaaggca accccgccag cctagccggg tcctcaacga caggagcacg atcatgcgca 3300

cccgtggggc cgccatgccg gcgataatgg cctgcttctc gccgaaacgt ttggtggcgg 3360

gaccagtgac gaaggcttga gcgagggcgt gcaagattcc gaataccgca agcgacaggc 3420

cgatcatcgt cgcgctccag cgaaagcggt cctcgccgaa aatgacccag agcgctgccg 3480

gcacctgtcc tacgagttgc atgataaaga agacagtcat aagtgcggcg acgatagtca 3540

tgccccgcgc ccaccggaag gagctgactg ggttgaaggc tctcaagggc atcggtcgag 3600

atcccggtgc ctaatgagtg agctaactta cattaattgc gttgcgctca ctgcccgctt 3660

tccagtcggg aaacctgtcg tgccagctgc attaatgaat cggccaacgc gcggggagag 3720

gcggtttgcg tattgggcgc cagggtggtt tttcttttca ccagtgagac gggcaacagc 3780

tgattgccct tcaccgcctg gccctgagag agttgcagca agcggtccac gctggtttgc 3840

cccagcaggc gaaaatcctg tttgatggtg gttaacggcg ggatataaca tgagctgtct 3900

tcggtatcgt cgtatcccac taccgagata tccgcaccaa cgcgcagccc ggactcggta 3960

atggcgcgca ttgcgcccag cgccatctga tcgttggcaa ccagcatcgc agtgggaacg 4020

atgccctcat tcagcatttg catggtttgt tgaaaaccgg acatggcact ccagtcgcct 4080

tcccgttccg ctatcggctg aatttgattg cgagtgagat atttatgcca gccagccaga 4140

cgcagacgcg ccgagacaga acttaatggg cccgctaaca gcgcgatttg ctggtgaccc 4200

aatgcgacca gatgctccac gcccagtcgc gtaccgtctt catgggagaa aataatactg 4260

ttgatgggtg tctggtcaga gacatcaaga aataacgccg gaacattagt gcaggcagct 4320

tccacagcaa tggcatcctg gtcatccagc ggatagttaa tgatcagccc actgacgcgt 4380

tgcgcgagaa gattgtgcac cgccgcttta caggcttcga cgccgcttcg ttctaccatc 4440

gacaccacca cgctggcacc cagttgatcg gcgcgagatt taatcgccgc gacaatttgc 4500

gacggcgcgt gcagggccag actggaggtg gcaacgccaa tcagcaacga ctgtttgccc 4560

gccagttgtt gtgccacgcg gttgggaatg taattcagct ccgccatcgc cgcttccact 4620

ttttcccgcg ttttcgcaga aacgtggctg gcctggttca ccacgcggga aacggtctga 4680

taagagacac cggcatactc tgcgacatcg tataacgtta ctggtttcac attcaccacc 4740

ctgaattgac tctcttccgg gcgctatcat gccataccgc gaaaggtttt gcgccattcg 4800

atggtgtccg ggatctcgac gctctccctt atgcgactcc tgcattagga agcagcccag 4860

tagtaggttg aggccgttga gcaccgccgc cgcaaggaat ggtgcatgca aggagatggc 4920

gcccaacagt cccccggcca cggggcctgc caccataccc acgccgaaac aagcgctcat 4980

gagcccgaag tggcgagccc gatcttcccc atcggtgatg tcggcgatat aggcgccagc 5040

aaccgcacct gtggcgccgg tgatgccggc cacgatgcgt ccggcgtaga ggatcgagat 5100

ctcgatcccg cgaaattaat acgactcact ataggggaat tgtgagcgga taacaattcc 5160

cctctagaaa taattttgtt taactttaag aaggagatat acatatgagc aagcgtaaac 5220

tgctgaagaa aatcgagaag aaagacacca gcagcgtgct gacccagaag aaacaaacca 5280

agaccctgtg ggcggatggc gtgcaggttg acgataagga cttcaaaccg agcaccgaaa 5340

actttggtac caactacctg gcggcggagt atggtatcgg caagggttac tacgatatca 5400

acaagaagtt cgacggcacc gacgatgacc tgtgcagcgg tgtggttgcg gcgaaccagc 5460

tgcactggtg gctggatcgt aacaaggact acattgaaaa atatcgtcag caaagcaagg 5520

ataacggcgt gaccatcggt aacaccgaca ttttcgaact gaacaaactg cacgatgagg 5580

accagagcaa cttctttgat ttcatcaaga aaagcttcgg caacaagttt ctgcaaccgg 5640

agcgtctgct gaacatgtac attaacggct acggttatct gaccagccag gataaggcga 5700

aaaccagcca accgagcccg agcaagctga acttctttca gaaggttttc aaaaacaacc 5760

tgctgaccga caaaaccccg atcaacgata ttgacgaatt tagcagccag accaagaacg 5820

cgctgcaaaa ccacaaagtg ctggcggtta gcttcgcgag catcaagaac cgtggcctgg 5880

gtcacgtggt taccgtgtgg ggcgcggatt ttgacgagaa cggtaaagtg gttgcgctgt 5940

atgttaccga cagcgatgac cgtagcaaga acattggcaa cgcgaaactg ggtatgaaga 6000

aactgcgtat cgaagtgagc gcgcaggata gcagcaccat taagctgacc ggcttcgagg 6060

acaaaaacag cggtggcagc ctgcgtcacc tgtacagcct gagcaccggt gaacaaatct 6120

ggaagaagta cttcgaggaa accgaaaagg agcgtattcg tctggaggaa gaggcggata 6180

aggcgaaact ggaacaggac cgtatccaaa aagaggcgga agagaaactg gcgctggaaa 6240

aggcggaaaa agagcgtatc cgtctggaag aggaagcgga caaagcgaag ctggaacaag 6300

accgtattca aaaagaggcg gaggaaaaac tggcgctgga gaaagcggag aaggaacgta 6360

tccgcctgga ggaagaggcg gacaaagcga aactggaaca agatcgcatt cagaaagagg 6420

cggaggagaa gctggcgctg gaaaaagcgg agaaggagcg cattcgcctg gaagaggaag 6480

cggataaagc gaagctggaa caggatcgca tccagaaaga ggcggaagag aagctggcgc 6540

tggagaaggc ggagaaggag cgtatccgac tggaggaaga ggcggcgaaa gctaaactgg 6600

aacaagaaaa acaaattgcg accgcgccgc agccggacaa gaaacaagaa aacaccacca 6660

gcgaacagga gaaaccggcg ccgaccgagc tgccgccgct ggttaacaaa gcggatgaaa 6720

ccgagacccc gcgtgagacc gcgccggacc aaaccccgag cgcgaccaac accttccgta 6780

agatcctgcc gaaaatgaac gctgtgagcc agttctttag ccaactgatg ggtaccatcc 6840

agattgtttt cgcgtttatc ctgaagattt ttaaaggcag cggtcatcac caccaccacc 6900

actaactcga gcaccaccac caccaccact gagatccggc tgctaacaaa gcccgaaagg 6960

aagctgagtt ggctgctgcc accgctgagc aataactagc ataacccctt ggggcctcta 7020

aacgggtctt gaggggtttt ttgctgaaag gaggaactat atccggat 7068

<210> 19
<211> 6327
<212> DNA
<213> Artificial Sequence

<220>
<223> vector

<400> 19
tggcgaatgg gacgcgccct gtagcggcgc attaagcgcg gcgggtgtgg tggttacgcg 60

cagcgtgacc gctacacttg ccagcgccct agcgcccgct cctttcgctt tcttcccttc 120

ctttctcgcc acgttcgccg gctttccccg tcaagctcta aatcgggggc tccctttagg 180

gttccgattt agtgctttac ggcacctcga ccccaaaaaa cttgattagg gtgatggttc 240

acgtagtggg ccatcgccct gatagacggt ttttcgccct ttgacgttgg agtccacgtt 300

ctttaatagt ggactcttgt tccaaactgg aacaacactc aaccctatct cggtctattc 360

ttttgattta taagggattt tgccgatttc ggcctattgg ttaaaaaatg agctgattta 420

acaaaaattt aacgcgaatt ttaacaaaat attaacgttt acaatttcag gtggcacttt 480

tcggggaaat gtgcgcggaa cccctatttg tttatttttc taaatacatt caaatatgta 540

tccgctcatg agacaataac cctgataaat gcttcaataa tattgaaaaa ggaagagtat 600

gagtattcaa catttccgtg tcgcccttat tccctttttt gcggcatttt gccttcctgt 660

ttttgctcac ccagaaacgc tggtgaaagt aaaagatgct gaagatcagt tgggtgcacg 720

agtgggttac atcgaactgg atctcaacag cggtaagatc cttgagagtt ttcgccccga 780

agaacgtttt ccaatgatga gcacttttaa agttctgcta tgtggcgcgg tattatcccg 840

tattgacgcc gggcaagagc aactcggtcg ccgcatacac tattctcaga atgacttggt 900

tgagtactca ccagtcacag aaaagcatct tacggatggc atgacagtaa gagaattatg 960

cagtgctgcc ataaccatga gtgataacac tgcggccaac ttacttctga caacgatcgg 1020

aggaccgaag gagctaaccg cttttttgca caacatgggg gatcatgtaa ctcgccttga 1080

tcgttgggaa ccggagctga atgaagccat accaaacgac gagcgtgaca ccacgatgcc 1140

tgcagcaatg gcaacaacgt tgcgcaaact attaactggc gaactactta ctctagcttc 1200

ccggcaacaa ttaatagact ggatggaggc ggataaagtt gcaggaccac ttctgcgctc 1260

ggcccttccg gctggctggt ttattgctga taaatctgga gccggtgagc gtgggtctcg 1320

cggtatcatt gcagcactgg ggccagatgg taagccctcc cgtatcgtag ttatctacac 1380

gacggggagt caggcaacta tggatgaacg aaatagacag atcgctgaga taggtgcctc 1440

actgattaag cattggtaac tgtcagacca agtttactca tatatacttt agattgattt 1500

aaaacttcat ttttaattta aaaggatcta ggtgaagatc ctttttgata atctcatgac 1560

caaaatccct taacgtgagt tttcgttcca ctgagcgtca gaccccgtag aaaagatcaa 1620

aggatcttct tgagatcctt tttttctgcg cgtaatctgc tgcttgcaaa caaaaaaacc 1680

accgctacca gcggtggttt gtttgccgga tcaagagcta ccaactcttt ttccgaaggt 1740

aactggcttc agcagagcgc agataccaaa tactgtcctt ctagtgtagc cgtagttagg 1800

ccaccacttc aagaactctg tagcaccgcc tacatacctc gctctgctaa tcctgttacc 1860

agtggctgct gccagtggcg ataagtcgtg tcttaccggg ttggactcaa gacgatagtt 1920

accggataag gcgcagcggt cgggctgaac ggggggttcg tgcacacagc ccagcttgga 1980

gcgaacgacc tacaccgaac tgagatacct acagcgtgag ctatgagaaa gcgccacgct 2040

tcccgaaggg agaaaggcgg acaggtatcc ggtaagcggc agggtcggaa caggagagcg 2100

cacgagggag cttccagggg gaaacgcctg gtatctttat agtcctgtcg ggtttcgcca 2160

cctctgactt gagcgtcgat ttttgtgatg ctcgtcaggg gggcggagcc tatggaaaaa 2220

cgccagcaac gcggcctttt tacggttcct ggccttttgc tggccttttg ctcacatgtt 2280

ctttcctgcg ttatcccctg attctgtgga taaccgtatt accgcctttg agtgagctga 2340

taccgctcgc cgcagccgaa cgaccgagcg cagcgagtca gtgagcgagg aagcggaaga 2400

gcgcctgatg cggtattttc tccttacgca tctgtgcggt atttcacacc gcatatatgg 2460

tgcactctca gtacaatctg ctctgatgcc gcatagttaa gccagtatac actccgctat 2520

cgctacgtga ctgggtcatg gctgcgcccc gacacccgcc aacacccgct gacgcgccct 2580

gacgggcttg tctgctcccg gcatccgctt acagacaagc tgtgaccgtc tccgggagct 2640

gcatgtgtca gaggttttca ccgtcatcac cgaaacgcgc gaggcagctg cggtaaagct 2700

catcagcgtg gtcgtgaagc gattcacaga tgtctgcctg ttcatccgcg tccagctcgt 2760

tgagtttctc cagaagcgtt aatgtctggc ttctgataaa gcgggccatg ttaagggcgg 2820

ttttttcctg tttggtcact gatgcctccg tgtaaggggg atttctgttc atgggggtaa 2880

tgataccgat gaaacgagag aggatgctca cgatacgggt tactgatgat gaacatgccc 2940

ggttactgga acgttgtgag ggtaaacaac tggcggtatg gatgcggcgg gaccagagaa 3000

aaatcactca gggtcaatgc cagcgcttcg ttaatacaga tgtaggtgtt ccacagggta 3060

gccagcagca tcctgcgatg cagatccgga acataatggt gcagggcgct gacttccgcg 3120

tttccagact ttacgaaaca cggaaaccga agaccattca tgttgttgct caggtcgcag 3180

acgttttgca gcagcagtcg cttcacgttc gctcgcgtat cggtgattca ttctgctaac 3240

cagtaaggca accccgccag cctagccggg tcctcaacga caggagcacg atcatgcgca 3300

cccgtggggc cgccatgccg gcgataatgg cctgcttctc gccgaaacgt ttggtggcgg 3360

gaccagtgac gaaggcttga gcgagggcgt gcaagattcc gaataccgca agcgacaggc 3420

cgatcatcgt cgcgctccag cgaaagcggt cctcgccgaa aatgacccag agcgctgccg 3480

gcacctgtcc tacgagttgc atgataaaga agacagtcat aagtgcggcg acgatagtca 3540

tgccccgcgc ccaccggaag gagctgactg ggttgaaggc tctcaagggc atcggtcgag 3600

atcccggtgc ctaatgagtg agctaactta cattaattgc gttgcgctca ctgcccgctt 3660

tccagtcggg aaacctgtcg tgccagctgc attaatgaat cggccaacgc gcggggagag 3720

gcggtttgcg tattgggcgc cagggtggtt tttcttttca ccagtgagac gggcaacagc 3780

tgattgccct tcaccgcctg gccctgagag agttgcagca agcggtccac gctggtttgc 3840

cccagcaggc gaaaatcctg tttgatggtg gttaacggcg ggatataaca tgagctgtct 3900

tcggtatcgt cgtatcccac taccgagata tccgcaccaa cgcgcagccc ggactcggta 3960

atggcgcgca ttgcgcccag cgccatctga tcgttggcaa ccagcatcgc agtgggaacg 4020

atgccctcat tcagcatttg catggtttgt tgaaaaccgg acatggcact ccagtcgcct 4080

tcccgttccg ctatcggctg aatttgattg cgagtgagat atttatgcca gccagccaga 4140

cgcagacgcg ccgagacaga acttaatggg cccgctaaca gcgcgatttg ctggtgaccc 4200

aatgcgacca gatgctccac gcccagtcgc gtaccgtctt catgggagaa aataatactg 4260

ttgatgggtg tctggtcaga gacatcaaga aataacgccg gaacattagt gcaggcagct 4320

tccacagcaa tggcatcctg gtcatccagc ggatagttaa tgatcagccc actgacgcgt 4380

tgcgcgagaa gattgtgcac cgccgcttta caggcttcga cgccgcttcg ttctaccatc 4440

gacaccacca cgctggcacc cagttgatcg gcgcgagatt taatcgccgc gacaatttgc 4500

gacggcgcgt gcagggccag actggaggtg gcaacgccaa tcagcaacga ctgtttgccc 4560

gccagttgtt gtgccacgcg gttgggaatg taattcagct ccgccatcgc cgcttccact 4620

ttttcccgcg ttttcgcaga aacgtggctg gcctggttca ccacgcggga aacggtctga 4680

taagagacac cggcatactc tgcgacatcg tataacgtta ctggtttcac attcaccacc 4740

ctgaattgac tctcttccgg gcgctatcat gccataccgc gaaaggtttt gcgccattcg 4800

atggtgtccg ggatctcgac gctctccctt atgcgactcc tgcattagga agcagcccag 4860

tagtaggttg aggccgttga gcaccgccgc cgcaaggaat ggtgcatgca aggagatggc 4920

gcccaacagt cccccggcca cggggcctgc caccataccc acgccgaaac aagcgctcat 4980

gagcccgaag tggcgagccc gatcttcccc atcggtgatg tcggcgatat aggcgccagc 5040

aaccgcacct gtggcgccgg tgatgccggc cacgatgcgt ccggcgtaga ggatcgagat 5100

ctcgatcccg cgaaattaat acgactcact ataggggaat tgtgagcgga taacaattcc 5160

cctctagaaa taattttgtt taactttaag aaggagatat acatatgagc aagcgtaaac 5220

tgctgaagaa aatcgagaag aaagacacca gcagcgtgct gacccagaag aaacaaacca 5280

agaccctgtg ggcggatggc gtgcaggttg acgataagga cttcaaaccg agcaccgaaa 5340

actttggtac caactacctg gcggcggagt atggtatcgg caagggttac tacgatatca 5400

acaagaagtt cgacggcacc gacgatgacc tgtgcagcgg tgtggttgcg gcgaaccagc 5460

tgcactggtg gctggatcgt aacaaggact acattgaaaa atatcgtcag caaagcaagg 5520

ataacggcgt gaccatcggt aacaccgaca ttttcgaact gaacaaactg cacgatgagg 5580

accagagcaa cttctttgat ttcatcaaga aaagcttcgg caacaagttt ctgcaaccgg 5640

agcgtctgct gaacatgtac attaacggct acggttatct gaccagccag gataaggcga 5700

aaaccagcca accgagcccg agcaagctga acttctttca gaaggttttc aaaaacaacc 5760

tgctgaccga caaaaccccg atcaacgata ttgacgaatt tagcagccag accaagaacg 5820

cgctgcaaaa ccacaaagtg ctggcggtta gcttcgcgag catcaagaac cgtggcctgg 5880

gtcacgtggt taccgtgtgg ggcgcggatt ttgacgagaa cggtaaagtg gttgcgctgt 5940

atgttaccga cagcgatgac cgtagcaaga acattggcaa cgcgaaactg ggtatgaaga 6000

aactgcgtat cgaagtgagc gcgcaggata gcagcaccat taagctgacc ggcttcgagg 6060

acaaaaacag cggtggcagc ctgcgtcacc tgtacagcct gagcaccggt gaacaaatct 6120

ggaagaagta cttcgaggaa acccatcacc accaccacca ctaactcgag caccaccacc 6180

accaccactg agatccggct gctaacaaag cccgaaagga agctgagttg gctgctgcca 6240

ccgctgagca ataactagca taaccccttg gggcctctaa acgggtcttg aggggttttt 6300

tgctgaaagg aggaactata tccggat 6327